# EUROPEAN PATENT APPLICATION

(11) **EP 2 022 772 A1**
(43) Date of publication of application: **11.02.2009**
(21) Application number: 07253120.5
(22) Date of filing: 09.08.2007
(51) Int. Cl.: C07C 2/76, C07C 6/08, C07C 9/00

(54) **Process for converting methane into liquid alkane mixtures**

(71) Applicant: BP OIL INTERNATIONAL LIMITED, Sunbury-on-Thames, Middlesex TW16 7BP (GB)
(72) Inventor: Basset, Jean-Marie, 69100 Villeurbanne (FR); Taoufik, Mostafa, 69100 Villeurbanne (FR); Thivolle-Cazat, Jean, 69100 Villeurbanne (FR); Chakka, Sudhakar, Naperville, Illinois 60563 (US)
(74) Representative: De Kezel, Eric

(57) **Abstract**

The present invention relates to a process for converting methane into a liquid mixture of (C₃₊) alkanes having 3 carbon atoms and more, preferably (C₄₊) alkanes having 4 carbon atoms and more, especially (C₅₊) alkanes having 5 carbon atoms and more. It comprises a stage (1) comprising converting methane into acetylene, performed by submitting methane to a thermal cracking reaction, so as to form a reaction mixture (M1) comprising acetylene and hydrogen, a stage (2) comprising converting acetylene into ethane, performed by contacting the acetylene or the reaction mixture (M1) previously obtained with hydrogen in the presence of a hydrogenation catalyst (C1), so as to form a reaction mixture (M2) comprising ethane, a stage (3) comprising simultaneous ethane self- and cross-metathesis reactions performed by contacting the ethane or the reaction mixture (M2) previously obtained with a metal catalyst (C2) capable of producing, in contact with alkane, reactions involving the splitting and recombining of C-C and/or C-H and/or C-metal bonds, so as to form a reaction mixture (M3) comprising methane and a mixture of the (C₃₊), preferably the (C₄₊), especially the (C₅₊) alkanes, and a stage (4) comprising fractionating the reaction mixture (M3) so as to separate and to isolate said mixture in liquid form. Preferably, the methane is also separated in stage (4) and recycled into stage (1) for methane conversion into acetylene

## Description

The present invention relates to a process for converting methane into liquid alkane mixtures.

Methane is present in very great amounts in natural gas which is widely distributed in the world, and natural gas deposit sites are often very far removed from one another and from the consumption areas. It follows that methane and generally natural gas from which methane can be derived, often have to be transported over long distances. It is however difficult to transport them because of their gaseous nature. Methane (or natural gas from which methane can be generally derived) can nevertheless be liquefied and transported more easily in liquid form. However, the energy and capital costs in achieving the liquefaction conditions and in then transporting the methane or the natural gas in liquid form are very high and may be often prohibitive as regards the exploitation of natural gas deposits, particularly ones of low capacity.

A solution would be to convert the methane (or the natural gas from which methane can be derived) into mixtures of higher alkanes which would be liquid under standard temperature and pressure conditions or conditions close to the latter, and which could therefore be easily transportable in liquid form. Such a conversion process would have to be simple and realisable at relatively low costs, enabling it to be installed directly on the natural gas deposit sites. This is the solution which the present invention proposes.

Processes for converting methane into liquid fuels by an oxidising route has already been known for a long time. The processes comprise first of all a reforming stage for converting the methane into a mixture of carbon monoxide and hydrogen, also called "synthesis gas" or "syngas", and then one or more stages for converting said mixture into gasoline and other easily transportable fuels. However, said processes require a great deal of energy and high investment in particular in order to achieve very high temperatures. These processes, in addition to being very expensive to build and to operate, also generate substantial quantities of green house causing carbon dioxide. Furthermore, the "carbon atom efficiency" of such processes is undesirably low.

In addition, International Patent Application WO 98/02244 discloses a process for converting alkanes into their higher and lower homologues. According to the process, one or more starting alkanes are reacted on a metal catalyst suitable for alkane metathesis and chosen in particular from metal hydrides and organometallic compounds fixed to a solid support. However, the process relates neither to the conversion of methane, nor to that of natural gas, but to that of alkanes having at least 2 carbon atoms. Various conversions are shown as examples, in particular those of ethane, propane, butane or isobutane into their higher and lower homologues.

International Patent Applications WO 01/04077 and WO 03/066552 disclose processes for preparing alkanes by reacting methane with at least one other starting alkane in the presence of an alkane metathesis metal catalyst particularly capable of producing, in contact with alkane, reactions involving the splitting and recombining of carbon-carbon and/or carbon-hydrogen and/or carbon-metal bonds, the metal catalyst being preferably chosen from the catalysts mentioned in International Patent Application WO 98/02244. Said reaction known under the term "methane-olysis" permits the preparation of at least one or two alkanes having a number of carbon atoms less than or equal to that of the starting alkane and at least equal to 2. Various methane-olysis reactions are shown as examples, in particular by reacting methane with ethane, propane or n-butane. It appears that methane-olysis of ethane leads to formation of methane and ethane, that is to say to formation of the starting products of the reaction. In addition, it is shown that methane-olysis of propane leads to formation of ethane, and that of n-butane permits ethane and propane to be prepared. However, the methane-olysis process involves the utilisation of methane necessarily with another starting alkane, and leads to the formation of alkanes whose number of carbon atoms is not higher than that of the starting alkane.

International Patent Application WO 03/104171 discloses a process for converting methane into ethane by contacting methane with an alkane metathesis metal catalyst chosen in particular from the catalysts mentioned in International Patent Application WO 98/02244. The process employs a methane coupling reaction, also called a methane homologation reaction, which produces ethane and hydrogen. In addition, it is suggested that the ethane thus prepared can then be used in other processes for upgrading of the ethane, such as dehydrogenation, catalytic cracking or thermal cracking, in order to prepare more particularly olefins, such as ethylene. International Patent Application WO 03/104171 does not envisage a process for the preparation of higher alkanes beyond ethane, such as liquid alkane mixtures.

International Patent Application WO 2006/060692 discloses supported metal clusters comprising one or more metals of Groups 4, 5 and/or 6 of the Periodic Table of the Elements, and which can be used as catalysts for alkane reactions. More particularly, the supported metal clusters are capable of being used to catalyze the metathesis (also referred to as disproportionation) of alkanes, conversions of alkanes with each other to give alkane products with molecular weights different from those of the starting alkanes, and conversion of methane to higher molecular-weight alkanes. The only alkane reactions described and shown as examples relate to a self-metathesis of ethane to give propane and methane, and a conversion of methane and n-butane (i.e. a methane-olysis of n-butane) to give propane and ethane. However, the Application neither discloses, nor suggests a process for converting methane (or natural gas from which methane can be derived) into liquid alkane mixtures. Indeed, it is shown that methane reacting with other starting alkane (e.g. n-butane) leads to formation of final alkanes (e.g. propane and ethane) with molecular weight lower than that of the starting alkane.

Furthermore, since a long time, various processes were known for converting methane or natural gas at high temperature into acetylene and then into liquid hydrocarbons, such as described in European Patent Application 1 140 738. However, the liquid hydrocarbons thus obtained comprise very substantial amounts of aromatic compounds.

Thus, it appears that none of the above-mentioned patent applications proposes a process for converting methane (or natural gas from which methane can be derived) into liquid alkane mixtures, in particular mixtures of higher alkanes capable more particularly of being easily transportable in liquid form, e.g. under standard temperature and pressure conditions or conditions close to the latter.

A process has now been found for converting methane (or natural gas from which methane can be derived) into liquid mixtures of higher alkanes capable in particular of being easily transportable in liquid form, e.g. under standard temperature and pressure conditions or conditions close to the latter. The process can produce liquid alkane mixtures, in particular liquid mixtures of (C₃₊) alkanes, i.e. alkanes having 3 carbon atoms and more (e.g. liquid mixtures of C₃ to C₁₅ alkanes), or preferably liquid mixtures of (C₄₊) alkanes, i.e. alkanes having 4 carbon atoms and more (e.g. liquid mixtures of C₄ to C₁₅ alkanes), or especially liquid mixtures of (C₅₊) alkanes, i.e. alkanes having 5 carbon atoms and more (e.g. liquid mixtures of C₅ to C₁₅ alkanes). The process has the advantage to produce liquid alkane mixtures preferably having a high degree of purity, for instance being free of alkenes, alkynes, aromatic compounds, carbon monoxide, carbon dioxide, sulfur- and/or nitrogen-containing products. It also has the advantage of being able to produce liquid alkane mixtures with a narrow distribution of the number of carbon atoms, in particular a distribution ranging for example from 3 to 15, preferably from 4 to 15, especially from 5 to 15 carbon atoms. The distribution of the number of carbon atoms can be very narrow, in particular ranging from 3 to 13, preferably from 4 to 13, especially from 5 to 13 carbon atoms. More particularly, the distribution of the number of carbon atoms can be even more narrow, in particular ranging from 3 to 10, preferably from 4 to 10, especially from 5 to 10 carbon atoms, or even from 3 to 8, preferably from 4 to 8, especially from 5 to 8 carbon atoms. The present invention has, in addition, the advantage of proposing a process by a non-oxidising route, of being simple and direct, and consequently of being relatively inexpensive, so that it can be directly used on the natural gas deposit sites where methane can be generally issued. Furthermore, one of the most important advantages of the process of the present invention relates to its potential for an extremely high "carbon atom efficiency" which can approach 95% or higher for converting methane (or natural gas from which methane can be derived) into liquid alkane mixtures which can be easily transportable.

More particularly, the present invention relates to a process for converting methane into a liquid mixture of (C₃₊) alkanes having 3 carbon atoms and more, preferably (C₄₊) alkanes having 4 carbon atoms and more, especially (C₅₊) alkanes having 5 carbon atoms and more, characterised in that it comprises:
- a stage (1) comprising converting methane into acetylene, carried out by submitting methane to a thermal cracking reaction, so as to form a reaction mixture (M1) comprising acetylene and hydrogen, and optionally by fractionating the reaction mixture (M1) so as to separate the acetylene,
- a stage (2) comprising converting acetylene into ethane, carried out by contacting the acetylene or the reaction mixture (M1) previously obtained with hydrogen in the presence of a hydrogenation catalyst (C1), so as to form a reaction mixture (M2) comprising ethane, and optionally by fractionating said reaction mixture (M2) so as to separate the ethane,
- a stage (3) comprising simultaneous ethane self- and cross-metathesis reactions, carried out by contacting the ethane or the reaction mixture (M2) previously obtained with a metal catalyst (C2) capable of producing, in contact with alkane, reactions involving the splitting and recombining of carbon-carbon and/or carbon-hydrogen and/or carbon-metal bonds, so as to form a reaction mixture (M3) comprising methane and a mixture of the (C₃₊), preferably the (C₄₊), especially the (C₅₊) alkanes, and
- a stage (4) comprising fractionating the reaction mixture (M3) so as to separate and to isolate the mixture of the (C₃₊), preferably the (C₄₊), especially the (C₅₊) alkanes, in liquid form.

The methane used in stage (1) of the present process, or the natural gas which can substitute the methane or from which methane can be derived and used in said stage (1), may comprise one or more poisons capable more particularly of deactivating catalysts, and optionally one or more impurities such as nitrogen (N₂). Thus, the process advantageously may comprise, prior to stage (1), a purification stage comprising removing from the methane or from the natural gas, one or more poisons capable of deactivating in particular the hydrogenation catalyst (C1) and/or the metal catalyst (C2) as employed in the process, and/or one or more impurities such as nitrogen (N₂). The purification stage can comprise more particularly removing one or more poisons chosen from water, carbon dioxide and sulfur compounds especially selected from hydrogen sulfide, carbonyl sulfide and mercaptans, and/or one or more impurities such as nitrogen (N₂). Any method can be used for removing these poisons and impurities.

More particularly, several natural gas deposits may comprise substantial concentrations of nitrogen (N₂) as impurity. Since nitrogen (N₂) is not anticipated to harm the hydrogenation catalyst (C1) and/or the metal catalyst (C2), it might not be necessary to remove the nitrogen (N₂) from the methane or from the natural gas prior to contacting these catalysts, in particular if the concentration of nitrogen is small, e.g. lower than 1% by volume of the total volume of the gas. However, it is advisable to remove nitrogen (N₂) from the methane or from the natural gas prior to contacting said methane or said natural gas with these catalysts.

Thus, water can be removed by subjecting the methane or the natural gas to a dehydration process, preferably chosen from cryogenic dehydration, dehydration by absorption and adsorptive dehydration. A cryogenic dehydration generally comprises cooling the wet methane or the wet natural gas until the components to be removed precipitate by condensation or formation of hydrates. Methanol, glycol or a paraffin solvent can be used during such a cryogenic dehydration. The reduction in temperature can be achieve e.g. by Joule-Thomson expansion.

Dehydration by absorption generally comprises a glycol absorption, wherein glycol such as mono-, di- or preferably triethylene glycol exhibits a high absorption capacity for water.

Adsorptive dehydration generally comprises contacting the methane or the natural gas with any adsorbent suitable for retaining water, preferably chosen from molecular sieves, silica gels and Na₂O-containing silica.

Sulfur compounds and carbon dioxide can be removed from the methane or from the natural gas by any process preferably chosen from physical absorption processes, chemical absorption processes, physical-chemical absorption processes, liquid oxidation processes, adsorption processes and membrane processes.

Generally, absorption processes comprise a gas scrubbing wherein the sour methane or the sour natural gas components are reversibly bound to a solvent by chemical and/or physical absorption. In regeneration step, the components are then desorbed unchanged and the solvent can be recycled to the scrubber. In physical absorption processes, carbon dioxide and hydrogen sulfide are generally physically dissolved in a solvent preferably chosen from N-methylpyrrolidone, a mixture of poly(ethylene glycol dimethyl ether), poly(ethylene glycol methyl isopropyl ether) and propylene carbonate. Chemical absorption processes generally comprise an alkanolamine scrubbing wherein aqueous solutions of monoethanolamine, diethanolamine, diisopropylamine, diglycolamine or methyldiethanolamine can be used as absorbents. Physical-chemical absorption processes generally comprise using a combination of solvents described previously in physical absorption and chemical absorption processes.

Liquid oxidation processes generally comprise the following steps: (i) absorption of hydrogen sulfide in an alkaline solution, e.g. a solution of NaHCO₃-Na₂CO₃, (ii) oxidation of dissolved hydrogen sulfide ions preferably by vanadates or iron(III) aminopolycarboxylic acid chelates, to elemental sulfur with simultaneously reduction of a chemical oxygen carrier, e.g. anthraquinonedisulfonic acids, (iii) reoxidation of the active component with air, and (iv) separation of elemental sulfur by filtration, centrifugation, flotation or sedimentation.

Adsorption processes for removal of hydrogen sulfide and carbon dioxide generally comprise contacting the methane or the natural gas with any adsorbent preferably chosen from activated charcoal, iron oxide, zinc oxide and zeolitic molecular sieves.

Membrane processes especially used for natural gas can be simultaneously performed for separation of carbon dioxide and methane, removal of carbon dioxide and hydrogen sulfide, dehydration and recovery of methane and optionally the rest of the natural gas essentially comprising a mixture of (C₂₊) alkanes having 2 carbon atoms and more, preferably a mixture of C₂ to C₆ alkanes (hereafter referred as the (C₂₋₆) alkanes) or a mixture of higher alkanes. Generally, membrane processes comprise the following steps:
(i) absorption from the gas phase into the membrane matrix, (ii) diffusion through the membrane, and (iii) desorption out the membrane into the gas phase. Membranes generally are polymer membranes preferably chosen from asymmetric cellulose acetate or triacetate, composite layers silicone/polysulfone, composite layers polyetherimide and composite layers silicone/polycarbonate. Any membrane process may be used to remove the nitrogen (N₂) from the methane or from the natural gas. Some membranes, are capable of separating more than one component, and therefore depending on the composition of the mixture comprising the methane or the natural gas, appropriate separation methods can be chosen.

The most important source of methane as used in stage (1) of the present process is the natural gas. Thus, the process can comprise a preliminary stage comprising fractionating the components of the natural gas, so as to separate and to isolate (a) the methane from (b) the rest of the natural gas essentially comprising a mixture of the (C₂₊) alkanes, preferably the (C₂₋₆) alkanes.

The natural gas which can substitute the methane or from which methane can be derived and used in stage (1) of the present process, can be any natural gas, such that exploited directly on a deposit site. It is generally formed of a mixture of alkanes comprising mainly methane and, in a weaker proportion, a mixture of the (C₂₊) alkanes, especially the (C₂₋₆) alkanes, e.g. ethane, propane, butanes, pentanes and hexanes, for example with the following percentages expressed in mole:
- methane from 75 to 99 %, preferably from 80 to 98 %, particularly from 85 to 97%,
- ethane from 1 to 15 % , preferably from 1 to 10 % , particularly from 1 to 7 % ,
- propane from 0.1 to 10 % , preferably from 0.1 to 5 %, particularly from 0.1 to 3 %,
- butanes (n-butane and isobutane) from 0 to 3 %, preferably from 0.01 to 2 % , particularly from 0.01 to 1 % ,
- pentanes (n-pentane and isopentane) from 0 to 2 %, preferably from 0.01 to 1 %, and
- hexane(s) from 0 to 1 %, preferably from 0.01 to 0.1%.

Sometimes, natural gas may also comprise traces of heptane(s) and/or higher alkanes.

Thus, the methane used in stage (1) of the present process can be previously separated from natural gas by fractionation in a preliminary stage. The fractionation may comprise separating and isolating from the natural gas (a) the methane and optionally (b) the rest of the natural gas essentially comprising a mixture of the (C₂₊) alkanes, preferably the C₂ to C₈, particularly the C₂ to C₇, especially the C₂ to C₆ alkanes (i.e. the (C₂₋₆) alkanes) of the natural gas. More particularly, the preliminary stage can be performed in various ways, either intermittently (or discontinuously), or preferably continuously. It can comprise one or more methods suitable for fractionation of the natural gas, preferably chosen from any methods or combinations of methods for fractionation of the natural gas, in particular for fractionating natural gas into (a) the methane and (b) the rest of the natural gas essentially comprising a mixture of the (C₂₊) alkanes. The methods of fractionation are preferably chosen from the following six types of fractionation:
- fractionation by change of physical state, preferably of gaseous/liquid phase of the natural gas, in particular by distillation or by condensation (or liquefaction), more particularly by partial condensation (or partial liquefaction), in particular by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, in particular by means of absorbing oil,
- fractionation by cryogenic expansion, in particular by means of expansion turbine, and
- fractionation by compression, preferably by means of gas compressor.

The preliminary stage can comprise combining at least two successive fractionations of an identical or different type, in particular chosen from the six above-mentioned types of fractionation.

Any type of fractionation that is useful in general for separating methane from natural gas, can be utilized in the preliminary stage.

In practice, the preliminary stage comprising a fractionation of the natural gas can be performed by change of the physical state, preferably of the gaseous/liquid phase of the natural gas, particularly by distillation or by condensation (or liquefaction), more particularly by partial condensation (or partial liquefaction), e.g. in at least one distillation/condensation column or tower, or in a distillation column reactor. The fractionation can be performed by refrigeration or cryogenic processes, preferably by cooling of the natural gas to a temperature at which the (C₂₊) alkanes, preferably the (C₂₋₆) alkanes condense, so as to condense the (C₂₊) alkanes, preferably the (C₂₋₆) alkanes, to separate and optionally to isolate them from.the.methane which is thus separated and isolated preferably in gaseous form.

In practice, the preliminary stage comprising a fractionation of the natural gas can also be performed by molecular filtration, in particular by passing the natural gas through at least one semi-permeable and selective membrane; so as to arrest and optionally to isolate the (C₂₊) alkanes, preferably the (C₂₋₆) alkanes, and to allow the methane to pass through and to be thus separated and isolated preferably in gaseous form.

The semi-permeable and selective membranes can be chosen from any membrane suitable for separating methane from natural gas, preferably membranes chosen from zeolite membranes, porous alumina membranes, ceramic membranes, flowing liquid membranes and supported liquid membranes.

In practice, the preliminary stage comprising a fractionation of the natural gas can also be performed by adsorption, in particular by passing the natural gas onto at least one molecular sieve or any other adsorbent, so as to retain methane (or the (C₂₊) alkanes), and to allow the rest of the natural gas comprising the (C₂₊) alkanes (or the methane) to pass through, and then by subjecting the methane (or the (C₂₊) alkanes) thus retained to at least one desorption step, preferably by the TSA ("Temperature Swing Adsorption") method or the PSA ("Pressure Swing Adsorption") method, so as to separate and to isolate the methane and optionally the (C₂₊) alkanes.

The adsorbents can be chosen from any adsorbent suitable for retaining methane or the (C₂₊) alkanes, preferably the (C₂₋₆) alkanes, preferably adsorbents chosen from molecular sieves, silica gels, activated charcoals and activated carbons.

In practice, the preliminary stage comprising a fractionation of the natural gas can also be performed by absorption, in particular by contacting natural gas with an absorption oil, e.g. in an absorption tower, so as to soak up the (C₂₊) alkanes, preferably the (C₂₋₆) alkanes, and to form an oil/(C₂₊) alkane mixture, and preferably by subjecting said oil/(C₂₊) alkane mixture, e.g. in a recovery tower, to a temperature above the boiling point of the (C₂₊) alkanes, but below that of the oil, so as to separate and optionally to isolate the (C₂₊) alkanes from the oil and from the methane which is thus separated and isolated preferably in gaseous form.

In practice, the preliminary stage comprising a fractionation of the natural gas can also be performed by a cryogenic expansion, in particular by dropping the temperature of the natural gas to a temperature preferably in a range of from -80 to -90°C, in particular around -85°C, more particularly by use of external refrigerants to cool the natural gas and then by means of an expansion turbine which rapidly expands the chilled natural gas and causes the temperature to drop significantly, so as to condense the (C₂₊) alkanes, preferably the (C₂₋₆) alkanes, to separate and optionally to isolate them in liquid form from the methane which is thus separated and isolated preferably in gaseous form.

In practice, the preliminary stage comprising a fractionation of the natural gas can also be performed by compression, preferably by compressing natural gas, e.g. by means of a gas compressor, so as to condense the (C₂₊) alkanes, preferably the (C₂₋₆) alkanes, and to separate and optionally to isolate them in liquid form from the methane which is thus separated and isolated preferably in gaseous form.

The process of the present invention comprises stage (1) for converting methane into acetylene. Generally, stage (2) is carried out by submitting methane to a thermal cracking reaction, so as to form a reaction mixture (M1) comprising acetylene and hydrogen, and optionally by fractionating the reaction mixture (M1) so as to separate the acetylene.

In the thermal cracking reaction of stage (1), methane can be used alone or substantially alone, e.g. in the form of a mixture of methane with 1% by mole or less of one or more other alkanes preferably selected from the (C₂₊) alkanes present in natural gas. In this case, methane can be obtained by fractionating natural gas and separating and isolating methane, in particular by submitting the natural gas to the above-mentioned preliminary stage.

Methane can be also used in stage (1) in the form of a mixture of methane, preferably in a major molar proportion, with one or more other alkane(s) preferably selected from the other alkanes present in natural gas, in particular the (C₂₊) alkanes of the natural gas, especially in molar proportions similar or identical to those existing in the natural gas. In this case, the mixture of methane with one or more other alkane(s) may comprise more than 50% and less than 99% by mole of methane, preferably from 60% to less than 99% by mole of methane, especially from 75% to 99% by mole of methane. More particularly, methane can be used in stage (1) in the form of natural gas.

The thermal cracking reaction of stage (1) can be performed by submitting methane (or natural gas) to a very high temperature, preferably a temperature higher than 730°C, in particular higher than 1,000°C, especially higher than 1,200°C. The thermal cracking reaction can be carried out with a very short reaction time (or residence time) at high temperature, with a reaction time selected preferably from 0.1 to 50 ms (millisecond), particularly from 0.1 to 30 ms, especially from 0.1 to 10 ms. The thermal cracking reaction is generally stopped by quenching the reaction mixture (M1) obtained to a temperature selected preferably from 50 to 300°C, particularly from 80 to 250°C, in particular with a quench rate selected from 10⁴ to 10⁸, preferably from 10⁵ to 10⁷ °C/s*.*

The production of the acetylene by the thermal cracking of methane is generally described by the following reversible reaction (1):

2 CH₄ → C₂H₂ + 3 H₂ (1)

The thermal cracking reaction is a very highly endothermic reaction (ΔH (298 K) = 376 kJ/mol). It can be performed in various ways, preferably by a high-temperature process selected from partial combustion processes, electrothermic processes and high-temperature pyrolysis processes. These high-temperature processes generally require a large amount of energy. They differ essentially only in the manner in which the necessary energy is generated and transferred. They can be performed intermittently (or discontinuously) or preferably continuously.

In a partial combustion process (also sometimes called "partial oxidation process"), part of the methane (or natural gas) is generally burnt so as to reach the reaction temperature and supply the heat of the cracking reaction. The methane (or natural gas) partial combustion involves a flame reaction with the methane (or natural gas) and oxygen preferably pre-mixed, the flame reaction being in particular described according to the following equations (2) and (3):

CH₄ + O₂ → CO + H₂ + H₂O (2)

CO + H₂O → CO₂ + H₂ (3)

Generally, the weight ratio of oxygen to methane (or oxygen to natural gas) used in the partial combustion process of stage (1) can be chosen in a range of from 0.3 to 0.8, preferably from 0.4 to 0.7. This range is particularly selected for determining the appropriate reaction temperature.

In practice, a partial combustion process in stage (1) generally comprises:
(i) preheating methane (or natural gas) and oxygen preferably separately, in particular at a temperature selected from 500 to 700°C,
(ii) passing the methane (or natural gas) and the oxygen thus preheated through a mixing zone and a burner into a cracking reaction zone wherein they are partially ignited, preferably at a temperature above 1,000°C, in particular above 1,200°C, e.g. in a range from 1,000 to 3,000°C, so as to form the reaction mixture (M1), and
(iii) quenching said reaction mixture (M1) when leaving the cracking reaction zone, particularly by water or preferably by a hydrocarbon liquid or an oil.

In such a partial combustion process; the residence time (or reaction time) of the methane (or natural gas) in the cracking reaction zone is generally very short and is preferably selected from 0.1 to 50 ms, especially from 0.1 to 30 ms or even from 0.1 to 10 ms.

When leaving the cracking reaction zone, the reaction mixture (M1) is generally quenched by water so as to reach a temperature preferably chosen from 50 to 100°C, especially from 80 to 90°C, or preferably by a hydrocarbon liquid or an oil so as to reach a temperature preferably chosen from 100 to 300°C, especially from 150 to 250°C. The quench rate can be very high so as to avoid forming a lot of by-products, and can be selected from 10⁴ to 10⁸ °C/s, preferably from 10⁵ to 10⁷ °C/s. Advantageously, quenching by a hydrocarbon liquid, preferably by a light hydrocarbon liquid, may be performed by a pre-quenching so as to produce additional acetylene and ethylene, in particular at an intermediate temperature chosen from 800 to 1,500°C.

The reaction mixture (M1) obtained by a partial combustion process essentially may comprise acetylene and hydrogen, and also generally ethylene, unreacted methane (or unreacted natural gas generally comprising the unreacted methane and the unreacted (C₂₊) alkanes), oxygen-containing products such as carbon monoxide, carbon dioxide and unreacted oxygen, and optionally soot or elemental carbon.

An electrothermic process (also sometimes called "electric-arc process") is generally preferred to a partial combustion process for performing stage (1), essentially because in an electrothermic process, methane (or natural gas) consumption can be greatly reduced, electrical energy is generally available under favourable and more economical conditions, and acetylene is produced with a higher yield and a very reduced amount of by-products, in particular free from oxygen-containing products such as carbon monoxide, carbon dioxide and oxygen.

An electrothermic process generally comprises an electric-arc burning in methane (or natural gas) and producing the cracking of the methane (or natural gas). Because of hydrogen formation during the electrothermic cracking reaction, the arc burns in a hydrogen atmosphere and can form an hydrogen plasma. The conductivity and the high rate of ion-electron recombination for hydrogen mean generally that arcs above a certain length cannot be operated with alternating current at normal frequency and high voltage. That is why, the electrothermic process is preferably performed with the help of a direct current.

In practice, an electrothermic process carried out in stage (1) generally comprises:
(i) injecting methane (or natural gas) into an arc furnace, preferably under an absolute pressure preferably chosen from 0.05 to 0.15 MPa, in particular from 0.10 to 0.14 MPa,
(ii) maintaining the methane (or natural gas) in the arc furnace during a residence time (or reaction time) selected from 0.05 to 50 ms, preferably from 0.1 to 30 ms, particularly from 0.1 to 10 ms, so as to form the reaction mixture (M1), and
(iii) quenching said reaction mixture (M1) when leaving the arc furnace, preferably with the help of water or a hydrocarbon liquid or an oil.

In such an electrothermic process, the arc furnace can comprise a cathode, a vortex chamber and an anode. Cathode and anode are generally water-jacketed tubes of carbon steel. During the process, an arc bums between cathode and anode generally with a length preferably chosen from 0.5 to 2 m, particularly from 1.0 to 1.5 m and with a current, in particular a direct current of an intensity preferably chosen from 500 to 3,000 A, particularly from 1,000 to 2,000 A. The cathode can be connected to the high-voltage side of a rectifier (e.g. chosen from 3 to 15 kV, preferably from 5 to 10 kV) and electrically isolated from the other parts of the furnace. In the arc furnace, the vortex chamber can be located between the cathode and the anode. The methane (or natural gas) can be injected into the vortex chamber tangentially, in particular at a specific velocity so as to stabilize the arc by creating a vortex. The arc can burn in a dead zone, and the striking points of the arc on the electrodes are generally forced into a rapid rotation, so that they only burn for fractions of milliseconds at one point, and temperatures can locally reach 5,000 to 30,000°C, preferably 15,000 to 25,000°C, particularly in the centre of the arc. Because of the tangential flow of the gas, the arc can be surrounded by a sharply decreasing coaxial temperature field, and thus the temperatures at the wall of the electrode can be only in a range of from 400 to 800°C, preferably from 500 to 700°C.

The residence time (or reaction time) of methane (or natural gas) in the arc furnace generally is very short and is preferably chosen from 0.1 to 50 ms, particularly from 0.1 to 30 ms, especially from 0.1 to 10 ms. At the end of the arc furnace, the reaction mixture (M1) thus produced can still be at a temperature in a range of from 1,200 to 3,000°C, preferably from 1,500°C to 2,500°C. Advantageously, the high heat content of the reaction mixture (M1) can be used for additional ethylene production, preferably by means of a pre-quench with a liquid hydrocarbon, and can help to decrease the temperature of the reaction mixture (M1) to a range preferably chosen from 600 to 1,800°C, in particular from 1,000 to 1,500°C. Because acetylene rapidly decomposes at high temperatures, the reaction mixture (M1) is preferably quenched immediately, in particular with the help of water or a hydrocarbon liquid or an oil, to a temperature chosen preferably from 50 to 300°C, in particular from 100 to 250°C, with a quench rate preferably selected from 10⁴ to 10⁸ °C/s, in particular from 10⁵ to 10⁷ °C/s.

The reaction mixture (M1) obtained by an electrothermic process essentially may comprise acetylene and hydrogen, and also generally ethylene, unreacted methane (or unreacted natural gas generally comprising the unreacted methane and the unreacted (C₂₊) alkanes) and optionally soot or elemental carbon.

Stage (1) may also be performed by a high-temperature pyrolysis process generally comprising a thermal cracking reaction carried out by contacting the methane (or natural gas) with a heat carrier preferably selected from refractory materials, hot combustion gases and superheated steam. A high-temperature pyrolysis process can be generally performed by an indirect heat transfer. Thus, for instance, the process can comprise (i) cracking methane (or natural gas) with a reaction time (or residence time) particularly selected from 0.1 to 50 ms, in a refractory oven previously heated by combustion gases to a temperature preferably selected from 1,000 to 3,000°C, so as to form the reaction mixture (M1), and (ii) quenching said reaction mixture (M1) outside said refractory oven to a temperature generally selected from 50 to 300°C, preferably with a quench rate selected from 10⁴ to 10⁸ °C/s. The process can also comprise (i) producing heat in a cracking reactor by burning residual cracked gas with the help of oxygen and a burner, so that the temperature of the cracking reactor can be preferably selected from 2,000 to 3,000°C and adjusted by steam injection, then (ii) introducing methane (or natural gas) into the cracking reactor thus pre-heated, so as to produce the reaction mixture (M1) at a temperature preferably selected from 1,300 to 2,500°C with a residence time (or reaction time) in particular selected from 0.1 to 50 ms, preferably from 0.5 to 30 ms, especially from 1 to 10 ms, and (iii) quenching said reaction mixture (M1) to a temperature preferably selected from 50 to 300°C, with a quench rate selected preferably from 10⁴ to 10⁸, particularly from 10⁵ to 10⁷ °C/s.

Generally, the reaction mixture (M1) comprises acetylene, hydrogen, and optionally ethylene and unreacted methane (or unreacted natural gas), and optionally by-products generally comprising oxygen-containing products particularly selected from carbon monoxide, carbon dioxide and optionally unreacted oxygen, depending on the type of process used for performing the thermal cracking reaction in stage (1). More particularly, the reaction mixture (M1) comprising acetylene, hydrogen and optionally ethylene and unreacted methane (or unreacted natural gas), preferably obtained in stage (1) by a thermal cracking reaction carried out by an electrothermic process, can be directly used in stage (2) for converting acetylene and optionally ethylene into ethane. This is particularly the case when the reaction mixture (M1) obtained e.g. by such an electrothermic process is free from oxygen-containing products, in particular free from carbon monoxide, carbon dioxide and oxygen.

Alternatively, the reaction mixture (M1) comprising acetylene, hydrogen and optionally ethylene and unreacted methane (or unreacted natural gas) can be submitted, prior to stage (2), to a fractionation comprising separating and preferably isolating the acetylene optionally in mixture with the ethylene and/or the unreacted methane (or the unreacted natural gas) and optionally with the hydrogen, particularly in molar proportions with regard to acetylene identical to or lower than those in said reaction mixture (M1). In addition, the above-mentioned reaction mixture (M1) may also be submitted to a fractionation comprising separating from said reaction mixture (M1) at least a portion of the unreacted methane (or the unreacted natural gas) which is then preferably recycled to the thermal cracking reaction in stage (1).

The reaction mixture (M1) comprising oxygen-containing products (e.g. selected from carbon monoxide, carbon dioxide and unreacted oxygen), preferably obtained in stage (1) by a thermal cracking reaction carried out by a partial combustion process, can be preferably submitted to a fractionation. In this case, the fractionation may essentially comprise separating and eliminating said oxygen-containing products from said reaction mixture (M1). The fractionation may furthermore comprise separating and preferably isolating the acetylene optionally in mixture with the ethylene and/or the unreacted methane (or the unreacted natural gas) and optionally with the hydrogen, particularly in molar proportions with regard to acetylene identical to or lower than those in said reaction mixture (M1). In a similar way, the fractionation may also comprise separating from the reaction mixture (M1) at least a portion of the unreacted methane (or the unreacted natural gas) which then is preferably recycled to the thermal cracking reaction of stage (1).

Thus, in stage (1), the fractionation of the reaction mixture (M1) can be performed in various ways, either intermittently (or discontinuously) or preferably continuously. It can comprise one or more methods suitable for fractionating the reaction mixture (M1) essentially into the acetylene optionally in mixture with the ethylene and/or the unreacted methane (or the unreacted natural gas generally comprising the unreacted methane and the unreacted (C₂₊) alkanes), and optionally with the hydrogen, particularly in molar proportions with regard to acetylene identical to or lower than those in said reaction mixture (M1), and preferably for separating and eliminating any oxygen-containing products.

More particularly, the fractionation of the reaction mixture (M1) can be carried out by one or more of the following methods of fractionation:
- fractionation by change of physical state, preferably of gaseous/liquid phase of the mixture, in particular by distillation or by condensation (or liquefaction), ore particularly by partial condensation ( or partial liquefaction), in particular by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, in particular by means of absorbing oil or solvent,
- fractionation by cryogenic expansion, in particular by means of expansion turbine, and
- fractionation by compression, preferably by means of gas compressor.

More generally, a fractionation of the reaction mixture (M1) can be performed by change of the physical state, preferably of the gaseous/liquid phase of the mixture, e.g. in at least one distillation/condensation column or tower, in particular by refrigeration or cryogenic processes. Preferably it can be performed by at least one cooling of the gaseous reaction mixture (M1) to at least one temperature at which at least one component of the mixture condenses and the rest of the mixture remains in gaseous form, so as to condense, to separate and to isolate said component(s) from said rest of the mixture.

Generally, a fractionation of the reaction mixture (M1) can also be performed by molecular filtration of the mixture, preferably by passing the reaction mixture (M1) through at least one semi-permeable and selective membrane, so as to arrest and to isolate at least one component of the mixture and to allow the rest of the mixture to pass through.

Generally, a fractionation of the reaction mixture (M1) can also be performed by adsorption, preferably by passing the reaction mixture (M1) onto at least one molecular sieve or any other adsorbent, so as to retain at least one component of the reaction mixture (M1) and to allow the rest of the mixture to pass through, and by submitting the component(s) thus retained to at least one desorption step, preferably by the TSA ("Temperature Swing Adsorption") method or the PSA ("Pressure Swing Adsorption") method, so as to isolate said component(s).

Generally, a fractionation of the reaction mixture (M1) can also be performed by absorption, preferably by contacting the reaction mixture (M1) with an absorption oil or a solvent, so as to soak up at least one component of the mixture, to form an oil (or solvent)/component mixture and to allow the rest of the mixture to be free and not absorbed, then by subjecting said oil(or solvent/component mixture, e.g. in a recovery tower, to a temperature above the boiling point of the component(s), but below that of the oil (or the solvent), so as to separate and to isolate said component(s) from the oil (or the solvent).

Generally, a fractionation of the reaction mixture (M1) can also be performed by cryogenic expansion, in particular by dropping the temperature of the gaseous reaction mixture (M1) to a temperature so as to condense at least one component of the mixture while maintaining the rest of the mixture in gaseous form, with the help of an expansion turbine rapidly expanding the gaseous mixture and causing a significant drop of the temperature.

Generally, a fractionation of the reaction mixture (M1) can also be performed by compression, in particular by compressing the gaseous reaction mixture (M1) e.g. by means of a gas compressor, so as to condense at least one component of the mixture, to separate and to isolate said component, and to allow the rest of the mixture to be maintained in gaseous form.

One of the preferred fractionation methods can be by change of physical state, preferably of gaseous/liquid phase of the mixture, in particular by condensation or partial condensation, or by cryogenic expansion of the mixture.

Another preferred fractionation method can be by absorption, in particular by means of an absorbing solvent, particularly when the reaction mixture (M1) is compressed, preferably under a partial pressure of acetylene below 0.14 MPa. The dissolved gas is generally recovered by depressurizing the solvent and by vapor stripping at higher temperatures. The absorbing solvent can be selected from N-methylpyrrolidone, methanol, ammonia and dimethylformamide. It can be recovered from the gas streams leaving the fractionation zone by water scrubbing and distillation.

The fractionation can also be performed advantageously by a double (or multiple) fractionation of the reaction mixture (M1), comprising a combination of two (or more) successive fractionations of an identical or different type, preferably chosen from the six above-mentioned types of fractionation.

Thus, the unreacted methane (or the unreacted natural gas generally comprising the unreacted methane and the unreacted (C₂₊) alkanes) can be at least partially separated and isolated from the reaction mixture (M1), and then be preferably recycled into stage (1). It is generally observed that at least a portion of the ethylene and/or of the unreacted methane (or the unreacted natural gas) is preferably maintained in mixture with the acetylene or in the reaction mixture (M1), so as to be further used in stage (2) for converting acetylene and optionally ethylene into ethane.

In addition, the fractionation may also comprise separating and preferably isolating the hydrogen which can be then used in one or more applications or stages, distinct or not from those of the present process. For example, the hydrogen thus isolated can be used as a reagent in stage (2) for the acetylene hydrogenation and/or as an agent for activation or regeneration of the metal catalyst (C2) employed in stage (3). The activation or the regeneration of the metal catalyst (C2) can be carried out by contacting the catalyst with the hydrogen, for example either in stage (3) for the simultaneous ethane self- and cross-metathesis reactions, or in a stage distinct and separate from stage (3). The hydrogen can also be used advantageously in other applications or stages distinct or not from the present process, preferably as a fuel for producing thermal and/or electrical energies, in particular as a fuel in hydrogen fuel cells for producing electrical energy. Such thermal and/or electrical energies can be directly employed in the present process, preferably in stage (1) for the thermal cracking reaction, especially the partial combustion process or the electrothermic process converting methane or natural gas into acetylene. It can also be employed as a fuel for automobile, or as a reagent in a chemical, petrochemical or refinery plant. It is remarkable to notice that hydrogen produced and isolated by the present process can generally supply a great part of all the energy needed for running said present process.

The process of the present invention also comprises stage (2) for converting acetylene into ethane. Generally, stage (2) is carried out by contacting the acetylene or the reaction mixture (M1) obtained in stage (1) with hydrogen in the presence of a hydrogenation catalyst (C1), so as to form a reaction mixture (M2) comprising ethane, and optionally by fractionating said reaction mixture (M2) so as to separate the ethane.

In stage (2), the acetylene can be used alone or substantially alone, e.g. in the form of a mixture of acetylene with 1% by mole or less of ethylene and/or one or more alkanes, preferably methane and/or higher alkanes in particular as present in the natural gas, especially the (unreacted) methane and the (unreacted) (C₂₊) alkanes of the natural gas present in the reaction mixture (M1).

The acetylene can also be advantageously used in stage (2) in the form of a mixture of acetylene, preferably in a major molar proportion, with ethylene and/or one or more alkanes, particularly with the ethylene and/or the unreacted methane (or the unreacted natural gas generally comprising the unreacted methane and the unreacted (C₂₊) alkanes) present in the reaction mixture (M1), especially in molar proportions with regard to acetylene identical to or lower than those in said reaction mixture (M1). In this case, the mixture of acetylene with ethylene and/or one or more alkanes may comprise more than 50% and less than 99% by mole of acetylene, preferably from 60% to less than 99% by mole of acetylene, particularly from 75% to less than 99% by mole of acetylene. It is observed that acetylene used in such a mixture is particularly advantageous in terms of the control of the temperature of the hydrogenation reaction(s) converting the acetylene and optionally the ethylene into ethane in stage (2).

The hydrogen can be used in stage (2) alone (or substantially alone), or in the form of a mixture of hydrogen with acetylene, in particular with the reaction mixture (M1) obtained in stage (1). Fresh additional hydrogen can be used in addition to the hydrogen present in the reaction mixture (M1).

Stage (2) essentially comprises contacting the acetylene or the reaction mixture (M1) with hydrogen in the presence of a hydrogenation catalyst (C1). The hydrogenation catalyst (C1) can be any hydrogenation catalyst preferably suitable for performing the hydrogenation reaction(s) of the acetylene into ethane, either in two steps, i.e. converting acetylene into ethylene, and then ethylene into ethane, e.g. according to the following equations (4) and (5), or in one step, i.e. converting acetylene directly into ethane, e.g. according to the following equation (6):

C₂H₂ + H₂ → C₂H₄ (4)

C₂H₄ + H₂ → C₂H₆ (5)

or

C₂H₂ + 2 H₂ → C₂H₆ (6)

Optionally, some C₄ hydrocarbons, particularly C₄ alkanes can be also formed as by-products, essentially by dimerization of ethylene.

The hydrogenation catalyst (C1) can be selected from supported metal catalysts and supported metal sulfide catalysts, preferably from supported transition metal catalysts and supported transition metal sulfide catalysts.

Generally, the supported transition metal catalyst may comprise one, two or more transition metals preferably selected from chromium, molybdenum, tungsten, rhenium, iron, ruthenium, osmium, cobalt, rhodium, iridium, nickel, palladium, platinum, silver, gold, copper and zinc, more particularly from chromium, rhenium, iron, ruthenium, cobalt, rhodium, iridium, nickel, palladium, platinum, silver, gold, and copper, especially from rhenium, rhodium, iridium, nickel, palladium and platinum. The supported transition metal catalyst may advantageously comprise two different transition metals selected from the above-mentioned list, for example supported bi-metallic catalysts selected from platinum-iridium and platinum-rhenium.

Generally, the supported transition metal sulfide catalyst may comprise one, two or more transition metal sulfides preferably selected from sulfides of chromium, molybdenum, tungsten, rhenium, iron, ruthenium, osmium, cobalt, rhodium, iridium, nickel, palladium, platinum, copper and zinc, particularly from sulfides of chromium, rhenium, iron, ruthenium, cobalt, rhodium, iridium, nickel, palladium, platinum and copper.

The supported metal or metal sulfide catalysts may also comprise at least one promoter or additive preferably selected from alkali metal oxides, alkaline earth metal oxides, transition metal oxides and lanthanide oxides, particularly selected from potassium oxide, magnesium oxide, calcium oxide, titanium oxide, niobium oxide and cerium oxide.

The hydrogenation catalyst (C1) generally is a heterogeneous catalyst, preferably a catalyst comprising a solid support. The support can be chosen from metal oxides, refractory oxides, metals, carbon and molecular sieves, preferably from silica, alumina, silica-alumina, aluminium silicate, carbon, zeolites, clays, titanium oxide, cerium magnesium oxide, niobium oxide, tantalum oxide and zirconium oxide, more particularly from silica, alumina, silica-alumina and carbon. Thus, the hydrogenation catalyst (C1) preferably is a supported transition metal catalyst selected from palladium supported on alumina, palladium supported on silica, palladium supported on silica-alumina (e.g. on pumice), palladium supported on carbon (e.g. on carbon black), platinum supported on alumina, platinum supported on silica, rhodium supported on silica, iridium supported on silica, nickel supported on silica, platinum-iridium supported on silica and platinum-rhenium supported on silica.

The hydrogenation catalyst (C1) generally comprise from 0.05 to 25 %, preferably from 0.1 to 10% by weight of one, two or more metals.

The hydrogenation catalyst (C1) can be prepared in various ways, in particular selected from the following methods: wet impregnation of a metal compound onto a support, decomposition and/or reduction of an organometallic compound onto a support, precipitation or ion-exchange of a metal compound onto a support and then reduction of the metal, and vapor condensation or sublimation of a metal onto a support. The preparation of the hydrogenation catalyst (C1) can be accomplished by any method known in the art, for example according to the method described by G. C. Bond, D. A. Dowden and N. Mackenzie in Trans. Faraday Soc. 54, 1537 (1958), or by R. Pestman, A. J. den Hartog and V. Ponec in Catalysis Letters 4 (1990) 287-298), or by S. Asplund in Journal of catalysis 158, 267-278 (1996).

In stage (2), the contacting can be advantageously performed in conditions suited to promoting the development of the acetylene hydrogenation reaction to the production of ethane. Thus, it can be performed at a temperature chosen from 0 to 400°C, preferably from 20 to 300°C. It can also be performed under an absolute total pressure chosen from 0.1 to 10 MPa, preferably from 0.1 to 1.0 MPa.

In stage (2), the contacting can be advantageously performed in the presence of one or more inert agents, either liquid or gaseous, in particular selected from inert gases and preferably from alkanes. The inert gas can be chosen from nitrogen, helium and argon. The alkane can be preferably chosen from methane, ethane and alkanes of the natural gas, particularly from the methane and the (C₂₊) alkanes of the natural gas, especially the unreacted methane or the unreacted natural gas (generally comprising the unreacted methane and the unreacted (C₂₊) alkanes) present on the reaction mixture (M1) obtained in stage (1). The presence of one or more alkanes in the hydrogenation reaction of stage (2) greatly improved the control of the temperature of said hydrogenation reaction. Thus, the contacting can be performed under a partial pressure of inert gas or alkane selected from 0.1 to 10 MPa, particularly from 0.1 to 1.0 MPa.

In stage (2), the contacting can be performed in various ways, either intermittently (or discontinuously) or preferably continuously. It can be performed in a reaction zone preferably comprising an efficient heat removal system, particularly a reactor selected from tubular (or multi-tubular) reactors, slurry reactors, fluidised bed reactors, mechanically agitated bed reactors, fluidised and mechanically agitated bed reactors, fixed bed reactors, moving bed reactors and circulating bed reactors. The hydrogenation catalyst (C1) is generally solid and can be present in a reactor in the form of a solid mixture with an inert solid agent in particular capable of promoting the heat removal, and for instance selected from silica, alumina and silica-alumina. It can be arranged outside or preferably inside the tube(s) of a tubular (or multi-tubular) reactor. It can also form the bed of a fluidised and/or mechanically agitated bed, a fixed bed, a moving bed or a circulating bed of one of the above-mentioned bed reactors. It can also be in form of solid particles maintained in slurry in a slurry reactor. In stage (2), the contacting can be performed by continuously introducing the methane or the reaction mixture (M1) with an hourly space velocity preferably selected from 10² to 10⁶ of volumes of gas per volume of the hydrogenation catalyst (C1) per hour, into a reaction zone containing said hydrogenation catalyst (C1).

The reaction mixture (M2) obtained in stage (2) essentially comprises ethane and optionally unreacted acetylene, ethylene and hydrogen, and optionally one or more other alkanes preferably selected from methane, butanes and natural gas (generally comprising methane and the (C₂₊) alkanes). It can be further used directly in stage (3). The above-mentioned reaction mixture (M2) can be also submitted, prior to stage (3), to a fractionation comprising separating and preferably isolating the ethane or a mixture of the ethane with one or more other alkanes in particular selected from methane and natural gas, and optionally with the hydrogen. The above-mentioned reaction mixture (M2) may be furthermore submitted, prior to stage (3), to a fractionation comprising separating from said reaction mixture (M2) the unreacted acetylene, ethylene and optionally hydrogen which can be preferably recycled in stage (2). The method of fractionation can be chosen from one or more of the above-mentioned six types of fractionation, as previously described in the preliminary stage or optionally in stage (1).

The process of the present invention then comprises a stage (3) wherein simultaneous ethane self- and cross-metathesis reactions are performed. More particularly, stage (3) comprises contacting the ethane or the reaction mixture (M2) previously obtained with a metal catalyst (C2) capable of producing, in contact with alkane, reactions involving the splitting and recombining of carbon-carbon and/or carbon-hydrogen and/or carbon-metal bonds, so as to form a reaction mixture (M3) comprising methane and a mixture of the (C₃₊), preferably the (C₄₊), especially the (C₅₊) alkanes.

The ethane used in stage (3) is the ethane or the reaction mixture (M2) previously obtained in stage (2). The ethane can be used alone or substantially alone, e.g. in the form of a mixture of ethane with 1% by mole or less of one or more alkanes, in particular one or more other higher alkanes preferably selected from the mixture of the (C₂₊) alkanes present in natural gas.

In stage (3), the ethane can be also used in the form of a mixture of ethane, preferably in a major molar proportion, with one or more other higher alkanes preferably selected from the other higher alkanes present in natural gas, in particular from the mixture of the (C₂₊) alkanes of the natural gas, especially in molar proportions with regard to ethane similar or identical to those existing in the natural gas. In this case, the mixture of ethane with one or more other higher alkanes may comprise more than 50% and less than 99% by mole of ethane, preferably from 60% to less than 99% by mole of ethane. More particularly, the ethane (or the reaction mixture (M2)) previously obtained in stage (2) can be used in stage (3) in mixture or in combination with the mixture of the (C₂₊) alkanes obtained by fractionation of natural gas in a preliminary stage (as previously described) from which methane can be derived and used in stage (1). Thus, the mixture of the (C₂₊) alkanes in particular obtained by fractionation of natural gas (such as described in the preliminary stage) can comprise the C₂ to C₈, preferably the C₂ to C₇, particularly the C₂ to C₆ alkanes, especially the (C₂₋₆) alkanes of the natural gas. In stage (3), the ethane (or the reaction mixture (M2)) previously obtained in stage (2) and the mixture of the (C₂₊) alkanes can be contacted either separately or simultaneously, preferably in the form of a pre-mixture, with the catalyst (C2).

The catalyst (C2) can be chosen from alkane metathesis catalysts. It can be chosen in particular from supported metal clusters and preferably from metal hydrides, organometallic compounds and organometallic hydrides, particularly supported on, and more specifically grafted onto a solid support.

The catalyst (C2) can be chosen from supported metal clusters comprising one or more metals of Groups 4, 5 and/or 6 of the Periodic Table of the Elements, preferably tantalum. The Table (here and hereafter) is that proposed by IUPAC in 1991 and, for example, published by CRC Press, Inc. (USA) in "CRC Handbook of Chemistry and Physics", 76th Edition, David R. Lide (1995-1996). The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or (more) fewer metal atoms of the metal cluster. The metal atom of the metal cluster can be bonded in addition to at least one hydrocarbon radical and/or to at least one hydrogen atom. The solid support can be chosen from a metal oxide, a refractory oxide, a molecular sieve, a zeolite and a metal phosphate, preferably silica. More particularly, the supported metal cluster can comprise tantalum (as a metal cluster) and silica (as a solid support), preferably bonding between tantalum and tantalum, and also bonding between tantalum and silica, in particular bonding between tantalum and oxygen on the silica surface. The catalyst (C2) can be chosen from the supported metal clusters described in International Patent Application WO 2006/060692, and prepared according to any of the methods described in said Application.

The catalyst (C2) is preferably chosen from metal hydrides, organometallic compounds and organometallic hydrides, particularly supported on, and more specifically grafted onto, a solid support. It can comprise at least one metal chosen from lanthanides, actinides and metals of Groups 2 to 12, preferably transition metals of Groups 3 to 12, more particularly Groups 3 to 10 of the Periodic Table of the Elements. In particular, the metal of the catalyst can be chosen from scandium, yttrium, lanthanum, titanium, zirconium, hafnium, vanadium, niobium, tantalum, chromium, molybdenum, tungsten, rhenium, ruthenium, osmium, nickel, iridium; palladium, platinum, cerium and neodymium. It can be chosen, preferably, from yttrium, titanium, zirconium, hafnium, vanadium, niobium, tantalum, chromium, molybdenum, tungsten, rhenium, ruthenium and platinum, and more especially from niobium, tantalum, molybdenum, tungsten and rhenium.

The catalyst (C2) can be chosen from metal hydrides, and preferably from organometallic compounds and organometallic hydrides, comprising at least one hydrocarbon radical (R), either saturated or unsaturated, linear or branched, having preferably from 1 to 20, in particular from 1 to 14 carbon atoms. The hydrocarbon radical (R) can be chosen from alkyl radicals, in particular either saturated or unsaturated, linear or branched, aliphatic or alicyclic, for example alkyl, alkylidene or alkylidyne radicals, particularly from C₁ to C₁₀, from aryl radicals, in particular from C₆ to C₁₂, and from aralkyl, aralkylidene or aralkylidyne radicals, particularly from C₇ to C₁₄. Thus, the metal atom of the catalyst can preferably be bonded to at least one hydrogen atom and/or to at least one hydrocarbon radical (R), in particular by a single, double or triple carbon-metal bond.

The catalyst (C2) preferably selected from metal hydrides, organometallic compounds and organometallic hydrides, can also comprise one or more ligands, such as "ancillary" ligands, preferably comprising at least one oxygen atom and/or at least one nitrogen atom, and chosen more particularly from oxo, alkoxo, aryloxo, aralkyloxo, nitrido, imido and amido ligands. Generally, by oxo, alkoxo, aryloxo, aralkyloxo, nitrido, imido and amido ligands are respectively meant:
- a divalent oxo radical of general formula: = O
- a monovalent alkoxo, aryloxo or aralkyloxo radical of general formula: -OR'
- a trivalent nitrido radical of general formula : ≡ N
- a divalent imido radical of general formula: = NR", and
- a monovalent amido radical of general formula: - NR¹R²,
general formulae in which O represents an oxygen atom, N represents a nitrogen atom, R' represents an hydrogen atom or a monovalent hydrocarbon radical selected respectively from alkyl, aryl and aralkyl radicals, R" represents an hydrogen atom or a monovalent hydrocarbon radical, and R¹ and R², being identical or different, represent an hydrogen atom or a monovalent hydrocarbon radical. More particularly, R', R", R¹ and R² can be a monovalent hydrocarbon radical, either saturated or unsaturated, linear or branched, comprising from 1 to 20, preferably from 1 to 14 carbon atoms, and particularly chosen from alkyl radicals, preferably from C₁ to C₁₀, aralkyl radicals, preferably from C₇ to C₁₄, and aryl radicals, preferably from C₆ to C₁₂.

Thus, when the catalyst (C2), preferably selected from metal hydrides, organometallic compounds and organometallic hydrides, comprises at least one of the above-mentioned ligands, the metal of the catalyst is bonded to at least one hydrogen atom and/or to at least one hydrocarbon radical (R), and in addition to at least one of these ligands, e.g. to the O atom of the oxo radical by a double bond, or to the O atom of the alkoxo, aryloxo or aralkyloxo radical (OR') by a single bond, or to the N atom of the nitrido radical by a triple bond, or to the N atom of the imido radical (NR") by a double bond, or to the N atom of the amido radical (NR¹R²) by a single bond. Generally, the presence of the oxo, alkoxo, aryloxo, aralkyloxo, nitrido, imido and/or amido ligands in the catalyst (C2) can favourably influence the behaviour of the catalyst in the simultaneous ethane self- and cross-metathesis reactions of stage (3).

In addition, the catalyst (C2) chosen from metal hydrides, organometallic compounds and organometallic hydrides is preferably supported on; or more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support is in particular chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be chosen, preferably, from silicas, aluminas, silica-aluminas and aluminium silicates, either simple or modified by other metals. The solid support can have a specific surface area (B.E.T.) measured according to the standard ISO 9277 (1995) which is chosen in a range of from 0.1 to 5000 m²/g, preferably from 1 to 3000 m²/g, particularly from 1 to 1000 m²/g. It is preferred to use a catalyst (C2) grafted onto a solid support and in which the metal atom can be bonded to at least one atom of hydrogen and/or to at least one hydrocarbon radical (R), and also to at least one of the atoms of the support, in particular to at least one atom of oxygen, sulfur, carbon or nitrogen of the support, in particular when the support is chosen from metal or refractory oxides, sulfides, carbides, nitrides and salts. The catalyst (C2) can be preferably a metal hydride or an organometallic hydride grafted onto a solid support, more particularly chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten grafted onto alumina, and rhenium hydrides or organometallic rhenium grafted onto silica.

The catalyst (C2) chosen from metal hydrides, organometallic compounds and organometallic hydrides can be in particular selected from the metal catalysts described in International Patent Applications WO 98/02244, WO 03/061823 and WO 2004/089541, and prepared according to any of the methods described in said Applications.

In stage (3), the ethane previously obtained in stage (2) and optionally in mixture or in combination with the mixture of the (C₂₊) alkanes of the natural gas, in particular the (C₂₋₆) alkanes obtained in the preliminary stage, is contacted with the catalyst (C2). The contacting can be performed in various ways. Particularly, the contacting with the catalyst (C2) can be performed first of all with the ethane, then with the mixture of the (C₂₊) alkanes, or conversely first of all with the mixture of the (C₂₊) alkanes, then with the ethane, or preferably simultaneously with the ethane and the mixture of the (C₂₊) alkanes, either pre-mixed or not.

In stage (3), it is observed that ethane can react with itself in contact with the catalyst (C2), and produce more particularly methane and propane by an ethane metathesis reaction, in particular by an ethane self-metathesis reaction, according to the following equation (7):

2 C₂H₆ → CH₄ + C₃H₈ (7)

It is furthermore observed that stage (3) can involve another ethane metathesis reaction, particularly a cross-metathesis reaction between the ethane and the propane previously obtained and optionally resulting from the mixture of the (C₂₊) alkanes used in mixture or in combination with the ethane. This reaction can produce especially methane and butane(s), in particular according to the following equation (8):

C₂H₆ + C₃H₈ → CH₄ + C₄H₁₀ (8)

It is furthermore observed that stage (3) can also involve other similar ethane metathesis reactions, in particular other similar cross-metathesis reactions between the ethane and successively higher alkanes having at least 4 carbon atoms, previously obtained by other preceding cross-metathesis reactions and also optionally resulting from the mixture of the (C₂₊) alkanes used in mixture or in combination with the ethane. These reactions can produce especially methane and higher alkanes having one additional carbon atom compared with the higher alkanes involved in the reactions, for example according to the following equations (9) to (12):

C₂H₆ + C₄H₁₀ → CH₄ + C₅H₁₂ (9)

C₂H₆ + C₅H₁₂ → CH₄ + C₆H₁₄ (10)

C₂H₆ + C₆H₁₄ → CH₄ + C₇H₁₆ (11)

C₂H₆ + C₇H₁₆ → CH₄ + C₈H₁₈ (12)

It is thus found that in the process of the invention, ethane appears to be an essential intermediate alkane which allows ultimately for methane or natural gas from which methane can be derived and use, to be easily converted into a mixture of the (C₃₊), preferably the (C₄₊), especially the (C₅₊) alkanes. It is remarkable to note that the present process can be carried out with a high overall yield, preferably at a very high carbon atom efficiency, particularly when the methane produced in stage (3) is separated and isolated from the reaction mixture (M3) and then is preferably recycled into stage (1) for methane conversion into acetylene. Metathesis reactions between the (C₃₊), preferably the (C₄₊), especially the (C₅₊) alkanes and other (C₃₊), (C₄₊) or (C₅₊) alkanes are also possible in stage (3) leading to formation of higher alkanes.

Stage (3) thus leads to the formation of a reaction mixture (M3) comprising methane, a mixture of the (C₃₊), preferably the (C₄₊), especially the (C₅₊) alkanes, and optionally unreacted ethane and where applicable propane and/or butanes, in particular when respectively the desired mixture of the (C₄₊) or of the (C₅₊) alkanes is preferably prepared, instead of the desired mixture of the (C₃₊) alkanes. Under the operating conditions of stage (3), the reaction mixture (M3) is generally obtained in gaseous form or in mixed gaseous/liquid form.

Stage (3) can be advantageously performed in conditions suited to promoting the development of the ethane metathesis reactions, such as those described above, in particular towards an optimum production of the desired mixture of the (C₃₊), preferably the (C₄₊), especially the (C₅₊) alkanes. Thus, it can be performed at a temperature chosen in a range of from 20 to 400°C, preferably 50 to 350°C, particularly 70 to 300°C, more particularly 100 to 250°C. It can also be performed under an absolute total pressure chosen in a range of from 0.1 to 50 MPa, preferably 0.1 to 30 MPa, particularly 0.1 to 20 MPa. It can also be performed in the presence of at least one inert agent, either liquid or gaseous, in particular of at least one inert gas preferably chosen from nitrogen, helium and argon. It can also be performed in the presence of hydrogen, preferably of the hydrogen of the reaction mixture (M1) in stage (1), in particular under a partial pressure of hydrogen chosen in a range of from 0.1 kPa to 10 MPa, preferably from 1 kPa to 1 MPa.

Stage (3) can be performed in various ways, either intermittently (or discontinuously) or preferably continuously, more particularly in a reaction zone (Z1) which can be either distinct and separate from a fractionation zone (F1) wherein stage (4) is performed, e.g. in at least one reactor, or arranged in a part of said fractionation zone (F1), e.g. in at least one distillation column reactor, such as described in American Patent US 4,242,530.

In stage (3), it is possible, for example, to add the ethane (optionally in mixture or in combination with the mixture of the (C₂₊) alkanes) to the catalyst (C2), or to add the catalyst (C2) to the ethane (optionally in mixture or in combination with the mixture of the (C₂₊) alkanes), or else to add the ethane (optionally in mixture or in combination with the mixture of the (C₂₊) alkanes) and the catalyst (C2) simultaneously into the reaction zone (Z1).

When the mixture of the (C₂₊), preferably the (C₂₋₆) alkanes separated and isolated from natural gas in the preliminary stage is involved in stage (3), it is possible to add the ethane and said mixture of the (C₂₊) alkanes either separately to the catalyst (C2) in two separate sections of the reaction zone (Z1), or successively in one order or another to the catalyst (C2) in the same reaction zone (Z1), or else preferably simultaneously to the catalyst (C2) in the same reaction zone (Z1), the ethane and said mixture of the (C₂₊) alkanes being pre-mixed or not. It is also possible, for example, to add the catalyst (C2) to the ethane and to the mixture of the (C₂₊) alkanes in two separate sections of the reaction zone (Z1). It is also possible to add successively one part of the catalyst (C2) first to the ethane, then another part of the catalyst (C2) to the resulting mixture, and to add the mixture of the (C₂₊) alkanes to the resulting new mixture, in the same reaction zone (Z1). It is also possible to perform said successive additions in a different order, for example: to add successively one part of the catalyst (C2) first to the mixture of the (C₂₊) alkanes, then another part of the catalyst (C2) to the resulting mixture, and to add the ethane to the new resulting mixture, in the same reaction zone (Z1). It is also possible to add the catalyst (C2) to the ethane and to the mixture of the (C₂₊) alkanes pre-mixed in the same reaction zone (Z2). It is also possible to add the catalyst (C2), the ethane and the mixture of the (C₂₊) alkanes simultaneously in the same reaction zone (Z1).

The reaction zone (Z1) can comprise at least one reactor chosen from static reactors, recycling reactors and dynamic reactors. For example, stage (3) can be performed in a static reactor containing fixed quantities of the catalyst (C2), of the ethane and optionally of the mixture of the (C₂₊) alkanes. Stage (3) can also be performed in a recycling reactor into which the ethane and/or at least one component of the reaction mixture (M3), e.g. methane, the mixture of the (C₃₊), preferably the (C₄₊), especially the (C₅₊) alkanes, and optionally unreacted ethane and where applicable propane and/or butanes, can be recycled preferably continuously. Stage (3) can also be performed in a dynamic reactor containing the catalyst (C2), preferably in the form of solid particles, through which the ethane and optionally the mixture of the (C₂₊) alkanes can pass or circulate, preferably continuously.

In practice, stage (3) can be performed in a reaction zone (Z1) comprising at least one reactor chosen from tubular (or multi-tubular) reactors, distillation column reactors, slurry reactors, fluidised bed reactors, mechanically agitated bed reactors, fluidised and mechanically agitated bed reactors, fixed bed reactors, moving bed reactors and circulating bed reactors. The catalyst (C2) is generally solid, and can optionally be mixed with an inert solid agent chosen in particular from silicas, aluminas, silica-aluminas and aluminium silicates, either simple or modified by other metals. It can be arranged inside the tube(s) of a tubular (or multi-tubular) reactor. It can also be arranged inside a distillation column reactor, wherein the catalyst is preferably a component of a distillation system functioning as both a catalyst and a distillation packing, i.e. a packing for a distillation column having both a distillation function as in stage (4) and a catalytic function as in stage (3): for example, rings, saddles, balls, irregular shapes, granulates, sheets, tubes, spirals, packed in bags, as described in American Patent US 4,242,530. The catalyst (C2) can also form the bed of a fluidised and/or mechanically agitated bed; a fixed bed, a moving bed or a circulating bed of said reactors. The ethane and optionally the mixture of the (C₂₊) alkanes can be introduced into one of said reactors, preferably continuously, and generally can pass or circulate preferably continuously in the form of a gaseous stream into the tube(s), or through the bed or the distillation packing of said reactors. In order to promote the development of the ethane metathesis reactions towards an optimum production of the desired mixture of the (C₃₊), preferably the (C₄₊), especially the (C₅₊) alkanes, stage (3) can advantageously be performed by withdrawing said desired mixtures and/or the methane out of the reaction zone (Z1), preferably continuously.

Thus, for example, stage (3) can be performed:
(i) by continuously introducing the ethane and optionally the mixture of the (C₂₊) alkanes pre-mixed or not, into the reaction zone (Z1) containing the catalyst (C2), so as to form the reaction mixture (M3) in particular comprising methane, the mixture of the (C₃₊), preferably the (C₄₊), especially the (C₅₊) alkanes, and optionally unreacted ethane and where applicable propane and/or butanes, and
(ii) by continuously withdrawing at least a part of the reaction mixture (M3) out of the reaction zone (Z1), so as to subject said part of the reaction mixture (M3) to stage (4) for separating the mixture of the (C₃₊), preferably the (C₄₊), especially the (C₅₊) alkanes from the reaction mixture (M3) and isolating said mixture from the rest of the reaction mixture (M3) which is preferably returned into the reaction zone (Z1), particularly in order to pursue the simultaneous ethane self- and cross-metathesis reactions.

The present process then comprises a stage (4) comprising fractionating the reaction mixture (M3) so as to separate and to isolate the mixture of the (C₃₊), preferably the (C₄₊), especially the (C₅₊) alkanes, in liquid form. Stage (4) can be performed in various ways, either intermittently, or discontinuously, or preferably continuously, in a fractionation zone (F1). It can comprise one or more methods of fractionation of the reaction mixture (M3), in particular chosen from any known methods or combinations of method for fractionation of such a mixture, preferably from the following six types of fractionation:
- fractionation by change of physical state, preferably of gaseous/liquid phase, in particular by distillation or by condensation (or liquefaction), more particularly by partial condensation (or partial liquefaction), in particular by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor, so as in particular to separate and to isolate from the mixture (M3) the mixture of the (C₃₊), preferably the (C₄₊), especially the (C₅₊) alkanes and optionally methane, unreacted ethane and where applicable propane and/or butanes.

Any method that is useful in general for separating the mixture of the (C₃₊), preferably the (C₄₊), especially the (C₅₊) alkanes from methane, optionally from ethane and where applicable from propane and/or butanes, can be utilized in stage (4).

Stage (4) can be performed in a fractionation zone (F1) which is either distinct and separate from the reaction zone (Z1) wherein stage (3) is performed, e.g. in one or more distillation/condensation columns or towers, or arranged in a part of said reaction zone (Z1), e.g. in at least one distillation column reactor, such as described in American Patent US 4,242,530.

Thus, a distillation column reactor can be advantageously used both for stages (3) and (4), and contain the metal catalyst (C2). More particularly, the metal catalyst (C2) can be arranged inside the distillation column reactor, as a component of a distillation system functioning as both a catalyst and a distillation packing. Thus, stages (3) and (4) can be simultaneously performed in at least one distillation column reactor comprising both a reaction zone and a fractionation zone. In the reaction zone, the contacting of the ethane and optionally the mixture of the (C₂₊) alkanes with the metal catalyst (C2) can be performed as in stage (3), so as to form the reaction mixture (M3) preferably in a gaseous/liquid form. Simultaneously, in the fractionation zone, the separation of the mixture of the (C₃₊), preferably the (C₄₊), especially the (C₅₊) alkanes from the reaction mixture (M3) can be performed as in stage (4), in particular by distillation or by condensation, more particularly by partial condensation, so as to isolate said mixture.

More generally, a fractionation of the reaction mixture (M3) can be performed by change of the physical state, preferably of the gaseous/liquid phase of the mixture, e.g. in at least one distillation/condensation column or tower, or in a distillation column reactor, in particular by refrigeration or cryogenic processes. Preferably it can be performed by at least one cooling of the reaction mixture (M3) to at least one temperature at which at least one component of the mixture condenses and the rest of the mixture remains in gaseous form, so as to condense, to separate and to isolate said comnonent(s) from said rest of the mixture.

Generally, a fractionation of the reaction mixture (M3) can also be performed by molecular filtration of the mixture, preferably by passing the reaction mixture (M3) through at least one semi-permeable and selective membrane, so as to arrest and to isolate at least one component of the mixture and to allow the rest of the mixture to pass through.

Generally, a fractionation of the reaction mixture (M3) can also be performed by adsorption, preferably by passing the reaction mixture (M3) onto at least one molecular sieve or any other adsorbent, so as to retain at least one component of the mixture and to allow the rest of the mixture to pass through, and by submitting the component(s) thus retained to at least one desorption step, preferably by the TSA or the PSA method, so as to isolate said component(s).

Generally, a fractionation of the reaction mixture (M3) can also be performed by absorption, in particular by contacting the reaction mixture (M3) with an absorbing oil, so as to soak up at least one component of the mixture, to form an oil/component mixture and to allow the rest of the mixture to be free and not absorbed, and then by subjecting said oil/component mixture to a temperature above the boiling point of the component(s), but below that of the oil, so as to separate and to isolate the component(s) from the oil.

Generally, a fractionation of the reaction mixture (M3) can also be performed by cryogenic expansion, in particular by dropping the temperature of the reaction mixture (M3) to a temperature so as to condense at least one component of the mixture while maintaining the rest of the mixture in gaseous form, with the help of an expansion turbine rapidly expanding the gaseous mixture and causing a significant drop of the temperature.

Generally, a fractionation of the reaction mixture (M3) can also be performed by compression, in particular by compressing the reaction mixture (M3), e.g. by means of a gas compressor, so as to condense at least one component of the mixture, to separate and to isolate it from the rest of the gaseous mixture.

For example, a fractionation of the reaction mixture (M3) can be performed by change of the gaseous/liquid phase of the mixture, e.g. in at least one distillation/condensation column or tower, or in a distillation column reactor, in particular by cooling of the reaction mixture (M3) to a temperature at which the mixture of the (C₃₊), preferably the (C₄₊), especially the (C₅₊) alkanes condenses, so as to condense said mixture, to separate and to isolate it in liquid form from the rest of the gaseous mixture comprising methane and optionally unreacted ethane and where applicable propane and/or butanes.

For example, a fractionation of the reaction mixture (M3) can also be performed by molecular filtration, in particular by passing the gaseous reaction mixture (M3) through at least one semi-permeable and selective membrane, so as to arrest and to isolate the mixture of the (C₃₊), preferably the (C₄₊), especially the (C₅₊) alkanes, and to allow the rest of the gaseous mixture comprising methane and optionally unreacted methane and where applicable propane and/or butanes to pass through.

The semi-permeable and selective membranes can be chosen from any membrane suitable for separating the mixture of the (C₃₊), preferably the (C₄₊), especially the (C₅₊) alkanes from methane, optionally from ethane and where applicable from propane and/or butanes. The semi-permeable and selective membranes can be preferably chosen from zeolite membranes, porous alumina membranes, ceramic membranes, flowing liquid membranes and supported liquid membranes.

For example, a fractionation of the reaction mixture (M3) can also be performed by adsorption, in particular by passing the gaseous reaction mixture (M3) onto at least one molecular sieve or any other adsorbent, so as to retain the mixture of the (C₃₊), preferably the (C₄₊), especially the (C₅₊) alkanes, and to allow the rest of the gaseous mixture comprising methane and optionally unreacted ethane and where applicable propane and/or butanes to pass through, and by submitting said mixture thus retained to at least one desorption step, preferably by the TSA or PSA method, so as to isolate it.

The adsorbents can be chosen from any adsorbent suitable for separating the mixture of the (C₃₊), preferably the (C₄₊), especially the (C₅₊) alkanes from methane, optionally from ethane and where applicable from propane and/or butanes. The adsorbents can be preferably chosen from molecular sieves, silica gels, activated charcoals and activated carbons.

For example, a fractionation of the reaction mixture (M3) can also be performed by absorption, preferably by contacting the gaseous reaction mixture (M3) with an absorption oil, e.g. in an absorption tower, so as to soak up the mixture of the (C₃₊), preferably the (C₄₊), especially the (C₅₊) alkanes, and to form an oil/(C₃₊), (C₄₊) or (C₅₊) alkane mixture, and then preferably by subjecting said oil/(C₃₊), (C₄₊) or (C₅₊) alkane mixture, e.g. in a recovery tower, to a temperature above the boiling point of the mixture of the (C₃₊), (C₄₊) or (C₅₊) alkanes but below that of the oil, so as to separate and to isolate said mixture of the (C₃₊), preferably the (C₄₊), especially the (C₅₊) alkanes from the oil and from the rest of the gaseous mixture comprising methane, unreacted ethane and where applicable propane and/or butanes.

For example, a fractionation of the reaction mixture (M3) can also be performed by cryogenic expansion, preferably by dropping the temperature of the gaseous reaction mixture (M3) in particular by use of external refrigerants to cool the gaseous reaction mixture and then by means of an expansion turbine which rapidly expands the chilled gaseous reaction mixture and causes the temperature to drop significantly, so as to condense the mixture of the (C₃₊), preferably the (C₄₊), especially the (C₅₊) alkanes, to separate and to isolate it from the rest of the gaseous mixture comprising methane, unreacted ethane and where applicable propane and/or butanes.

For example, a fractionation of the reaction mixture (M3) can also be performed by compression, preferably by compressing the gaseous reaction mixture (M3), e.g. by means of a gas compressor, so as to condense the mixture of the (C₃₊), preferably the (C₄₊), especially the (C₅₊) alkanes, to separate and to isolate it in liquid form from the rest of the gaseous mixture comprising methane and optionally unreacted ethane and where applicable propane and/or butanes.

Stage (4) can also be performed advantageously by a double (or multiple) fractionation of the reaction mixture (M3), comprising a combination of two (or more) successive fractionations of an identical or different type, preferably chosen from the six above-mentioned types of fractionation, so as in particular to separate and to isolate (a) the mixture of the (C₃₊), preferably the (C₄₊), especially the (C₅₊) alkanes, (b) the methane and (c) unreacted ethane and where applicable unreacted propane and/or butanes.

The methane can be advantageously separated and isolated in stage (4), e.g. by the double (or multiple) fractionation, and then recycled into stage (1) for the methane conversion into acetylene. The unreacted ethane and/or where applicable the unreacted propane and/or butanes is/are also advantageously separated and optionally isolated in stage (4), e.g. by the double (or multiple) fractionation, and then preferably is/are recycled into stage (3) for the simultaneous ethane self- and cross-metathesis reactions, in particular together or separately.

The separation, isolation and recycling of the methane are particularly advantageous in the process of the present invention, since the methane is produced in large quantities in stage (3), and its recycling into stage (1) for the methane conversion into acetylene improves enormously the overall yield of the process. The same applies to the separation, isolation and recycling of the unreacted ethane and where applicable of the unreacted propane and/or butanes, since their recycling into stage (3) for the simultaneous ethane self- and cross-metathesis reactions improves substantially the output of said stage.

For example, it is possible to perform a double fractionation by subjecting the reaction mixture (M3) comprising the methane, the mixture of the (C₃₊), preferably the (C₄₊), especially the (C₅₊) alkanes, the unreacted ethane and where applicable the unreacted propane and/or butanes, to a combination of two successive fractionations of one and the same type, namely by change of the physical state, preferably of the gaseous/liquid phase of the mixture, e.g. in at least one or more distillation/condensation columns or towers, or in a distillation column reactor. Thus, the two successive fractionations can be performed:
(i) by a first cooling of the gaseous reaction mixture (M2) to a temperature at which the mixture of the (C₃₊), preferably the (C₄₊), especially the (C₅₊) alkanes condenses, so as to condense said mixture, to separate and to isolate it in liquid form from the rest of the gaseous mixture comprising the methane, the unreacted ethane and where applicable the unreacted propane and/or butanes, and
(ii) by a second cooling of said rest of the gaseous mixture to a temperature at which the unreacted ethane and where applicable the unreacted propane and/or butanes condense, so as to condense said unreacted ethane and where applicable said unreacted propane and/or butanes, to separate and to isolate it (or them) in liquid form from the methane which is preferably isolated in its turn in gaseous form.

For example, it is also possible to perform a double fractionation by subjecting the above-mentioned gaseous reaction mixture (M3) to a combination of two successive fractionations of one and the same type, namely by molecular filtration:
(i) by passing the gaseous reaction mixture (M3) through a first semi-permeable and selective membrane, so as to arrest and to isolate the mixture of the (C₃₊), preferably the (C₄₊), especially the (C₅₊) alkanes, and to allow the rest of the gaseous mixture comprising the methane, the unreacted ethane and where applicable the unreacted propane and/or butanes to pass through, and
(ii) by passing said rest of the gaseous mixture through a second semi-permeable and selective membrane; so as to arrest and to isolate the unreacted ethane and where applicable the unreacted propane and/or butanes, and to allow the methane to pass through and preferably to be isolated in its turn in gaseous form.

For example, it is also possible to perform a double fractionation by subjecting the above-mentioned gaseous reaction mixture (M3) to a combination of two successive fractionations of one and the same type, namely by adsorption:
(i) by passing the gaseous reaction mixture (M3) onto a first molecular sieve or any other adsorbent, so as to retain the mixture of the (C₃₊), preferably the (C₄₊), especially the (C₅₊) alkanes, and to allow the rest of the gaseous mixture comprising the methane, the unreacted ethane and where applicable the unreacted propane and/or butanes to pass through, then
(ii) by passing said rest of the gaseous mixture onto a second molecular sieve or any other adsorbent, so as to retain the unreacted ethane and where applicable the unreacted propane and/or butanes, and to allow the methane to pass through and preferably to be isolated, and
(iii) by respectively submitting the mixture of the (C₃₊), preferably the (C₄₊), especially the (C₅₊) alkanes and the unreacted ethane and where applicable the unreacted propane and/or butanes thus retained respectively to desorption steps, in particular by the TSA or PSA method, so as to isolate said mixture and said unreacted ethane and where applicable said unreacted propane and/or butanes.

For example, it is also possible to perform a double fractionation by subjecting the above-mentioned gaseous reaction mixture (M3) to a combination of two successive fractionations of one and the same type, namely by absorption, e.g. in at least one or two absorption towers:
(i) by contacting the gaseous reaction mixture (M3) with a first absorbing oil, so as to soak up the mixture of the (C₃₊), preferably the (C₄₊), especially the (C₅₊) alkanes, to form an oil/(C₃₊), (C₄₊) or (C₅₊) alkane mixture, and to allow the rest of the gaseous mixture comprising the methane, the unreacted ethane and where applicable the unreacted propane and/or butanes to be free in gaseous form,
(ii) by contacting said rest of the gaseous mixture with a second absorbing oil, so as to soak up the unreacted ethane and where applicable the unreacted propane and/or butanes, to form an oil/ethane, propane and/or butane mixture, and to allow the methane to be free in gaseous form and preferably to be separated and isolated, and
(iii) by subjecting said oil/(C₃₊), (C₄₊) or (C₅₊) alkane mixture, e.g. in a recovery tower, to a temperature above the boiling point of said (C₃₊), (C₄₊) or (C₅₊) alkane mixture, but below that of the oil, so as to separate and.to isolate said (C₃₊), (C₄₊) or (C₅₊) alkane mixture from the oil, and preferably by subjecting said oil/ethane, propane and/or butane mixture, e.g. in a recovery tower, to a temperature above the boiling point of the ethane and where applicable of the propane and/or the butanes, but below that of the oil, so as to separate and to isolate the unreacted ethane and where applicable the unreacted propane and/or butanes.

For example, it is also possible to perform a double fractionation by subjecting the above-mentioned gaseous reaction mixture (M3) to a combination of two successive fractionations of one and the same type, namely by cryogenic expansion:
(i) by dropping the temperature of the gaseous reaction mixture (M3) by means of an expansion turbine, so as to condense the mixture of the (C₃₊), preferably the (C₄₊), especially the (C₅₊) alkanes, to separate and to isolate it in liquid form from the rest of the gaseous mixture comprising the methane, the unreacted ethane and where applicable the unreacted propane and/or butanes, and
(ii) by dropping the temperature of said rest of the gaseous mixture by means of an expansion turbine, so as to condense the unreacted ethane and where applicable the unreacted propane and/or butanes, to separate and to isolate it (or them) in liquid form from the methane which is preferably isolated in its turn in gaseous form.

For example, it is also possible to perform a double fractionation by subjecting the above-mentioned gaseous reaction mixture (M3) to a combination of two successive fractionations of one and the same type, namely by compression:
(i) by compressing the gaseous reaction mixture (M3), e.g. by means of a gas compressor, so as to condense the mixture of the (C₃₊), preferably the (C₄₊), especially the (C₅₊) alkanes, to separate and to isolate it in liquid form from the rest of the gaseous mixture comprising the methane, the unreacted ethane and where applicable the unreacted propane and/or butanes, and
(ii) by compressing said rest of the gaseous mixture; e.g. by means of a gas compressor, so as to condense the unreacted ethane and where applicable the unreacted propane and/or butanes, to separate and to isolate it (or them) in liquid form from the methane which is preferably isolated in its turn in gaseous form.

It is also possible to perform a double (or multiple) fractionation by subjecting the above-mentioned reaction mixture (M3) to a combination of two successive fractionations of a different type, preferably chosen from the six above-mentioned types of fractionation.

More particularly, a double fractionation of the above-mentioned reaction mixture (M3) can be performed by combining two successive fractionations of a different type, namely:
- the one by change of the physical state, preferably of the gaseous/liquid phase of the mixture, in particular by distillation or by condensation, and the other by molecular filtration, preferably by means of semi-permeable and selective membrane, or
- the one by change of the physical state, preferably of the gaseous/liquid phase of the mixture, in particular by distillation or by condensation, and the other by adsorption, preferably by means of molecular sieve or any other adsorbent, or
- the one by change of the physical state, preferably of the gaseous/liquid phase of the mixture, in particular by distillation or by condensation, and the other by absorption, preferably by means of an absorbing oil, or
- the one by change of the physical state, preferably of the gaseous/liquid phase of the mixture, in particular by distillation or by condensation, and the other by cryogenic expansion, preferably by means of an expansion turbine, or
- the one by change of the physical state, preferably of the gaseous/liquid phase of the mixture, in particular by distillation or by condensation, and the other by compression, preferably by means of gas compressor, or
- the one by molecular filtration, preferably by means of a semi-permeable and selective membrane, and the other by adsorption, preferably by means of a molecular sieve or any other adsorbent, or
- the one by molecular filtration, in particular by means of a semi-permeable and selective membrane, and the other by absorption, preferably by means of an absorbing oil, or
- the one by molecular filtration, preferably by means of a semi-permeable and selective membrane, and the other by cryogenic expansion, preferably by means of an expansion turbine, or
- the one by molecular filtration, preferably by means of a semi-permeable and selective membrane, and the other by compression, preferably by means of a gas compressor, or
- the one by adsorption, preferably by means of a molecular sieve or any other adsorbent, and the other by absorption, preferably by means of an absorbing oil, or
- the one by adsorption, preferably by means of a molecular sieve or any other adsorbent, and the other by cryogenic expansion, preferably by means of an expansion turbine, or
- the one by adsorption, preferably by means of a molecular sieve or any other adsorbent, and the other by compression, preferably by means of a gas compressor, or
- the one by absorption, preferably by means of an absorbing oil, and the other by cryogenic expansion, preferably by means of an expansion turbine, or
- the one by absorption, preferably by means of an absorbing oil, and the other by compression, preferably by means of a gas compressor, or
- the one by cryogenic expansion, preferably by means of an expansion turbine, and the other by compression, preferably by means of a gas compressor.

Each of said combinations can be performed in a chronological order or in a reverse order.

Thus, for example, the two successive fractionations of a different type can be performed advantageously:
(i) by cooling the gaseous reaction mixture (M3) to a temperature at which the mixture of the (C₃₊), preferably the (C₄₊), especially the (C₅₊) alkanes condenses, so as to condense said mixture, to separate and to isolate it in liquid form from the rest of the gaseous mixture comprising the methane, the unreacted ethane and where applicable the unreacted propane and/or butanes, and
(ii) by passing said rest of the gaseous mixture through a semi-permeable and selective membrane, so as to arrest and to isolate the unreacted ethane and where applicable the unreacted propane and/or butanes, and to allow the methane to pass through and preferably to be isolated in its turn in gaseous form.

For example, it is also possible to perform said two successive fractionations of a different type, but in a reverse order, that is to say:
(i) by passing the gaseous reaction mixture (M3) through a semi-permeable and selective membrane, so as to arrest and to isolate the mixture of the (C₃₊), preferably the (C₄₊), especially the (C₅₊) alkanes, and to allow the rest of the gaseous mixture comprising the methane, the unreacted ethane and where applicable the unreacted propane and/or butanes to pass through, and
(ii) by cooling the rest of the gaseous mixture to a temperature at which the ethane and where applicable the propane and/or the butanes condense, so as to condense the unreacted ethane and where applicable the unreacted propane and/or butanes, to separate and to isolate it (or them) in liquid form from the methane which is preferably isolated in its turn in gaseous form.

Thus, by means of a double (or multiple) fractionation such as one of those described above, the methane thus isolated in stage (4) is preferably recycled into stage (1) for the methane conversion. In addition, the unreacted ethane and/or where applicable the unreacted propane and/or butanes thus isolated in stage (4) is/are preferably recycled into stage (3) for the simultaneous ethane sel- and cross-metathesis reactions, in particular together or separately.

The process of the invention has the advantage of converting methane or natural gas from which methane can be derived and used, easily and with a high yield into a liquid mixture of the (C₃₊), preferably the (C₄₊), especially the (C₅₊) alkanes which is capable of being easily transportable in liquid form, in conditions relatively close to standard temperature and pressure conditions. In addition, the liquid mixture thus obtained preferably contains substantially no aromatic compounds. The process of the present invention also exhibits a very high "carbon atom efficiency" which can be equal to or higher than 95%. Furthermore, it can advantageously produce great amounts of hydrogen which can be isolated and then supply sufficient thermal and/or electrical energies for running the present process.

The following examples illustrate the present invention.

### Example 1

### (1) Preparation of hydrogenation catalyst (C1): palladium supported on silica.

A hydrogenation catalyst (C1) comprising 1 wt.% Pd/SiO₂ was prepared by ion-exchange method using Pd(NH₃)₄(OH)₂ as a Pd precursor. Silica (Aerosil Degussa, 200 m²/g) was added to a solution of Pd(NH₃)₄(OH)₂ and the suspension was stirred for 2 hours before centrifuging. The catalyst cake thus obtained was washed in water, dried at 110°C overnight, calcined in air at 300°C for 2 hours, and then reduced in hydrogen (H₂) at 300°C for 1 hour before use in the conversion process.

### (2) Preparation of metal catalyst (C2): an organometallic tungsten hydride grafted onto alumina.

In a first stage, 2 g of an α-alumina containing 90 wt % of alumina and 9 wt % of water, and sold by Johnson Matthey (Great Britain) were subjected to a calcination treatment under a dry air current at 500°C for 15 hours, then to a dehydroxylation treatment under an absolute pressure of 10⁻² Pa, at 500°C, for 15 hours. At the end of this time, the alumina thus treated was cooled under an argon atmosphere to 25°C.

In a second stage, 2 g of the alumina prepared beforehand were isolated and introduced under an argon atmosphere into a reactor fitted with an agitator at 25°C. Then, 305 mg of a precursor (Pr'), namely tungsten tris(neopentyl)neopentylidyne of the general formula (13):

W [ - CH₂ - C(CH₃)₃]₃ [≡ C - C(CH₃)₃] (13)

were introduced into the reactor. Then, the reactor was heated to 66°C, and the mixture thus obtained was agitated in the dry state for 4 hours. At the end of this time, the reactor was cooled to 25°C, and the solid mixture thus obtained was then washed with n-pentane at 25°C. The solid compound thus washed was vacuum dried and then isolated under an argon atmosphere, so as to obtain an organometallic tungsten compound grafted onto alumina and containing 3.9 wt % of tungsten.

In a third stage, 40 mg of the organometallic tungsten compound grafted onto alumina prepared beforehand were isolated in a reactor and subjected to a hydro-genolysis treatment, by contacting said compound with hydrogen (H₂) under an absolute hydrogen pressure of 73 kPa, at 150°C, for 15 hours. At the end of this time, the reactor was cooled to 25°C, and there was obtained and isolated under an argon atmosphere a metal catalyst (C2) based on an organometallic tungsten hydride grafted onto alumina which contained 3.9 wt % of tungsten.

### (3) Conversion of methane into a liquid mixture of the (C₃₊) alkanes.

In stage (1) of the process, methane was converted into acetylene by a thermal cracking reaction carried out by an electrothermic process using an arc furnace comprising a cathode, an anode and a vortex chamber located between the cathode and the anode. Methane was continuously introduced tangentially into the vortex chamber of the are furnace, so as to create a vortex and to stabilize the arc. The arc burnt between the cathode and the anode with a current of 1,200 A. The cathode was connected to the high-voltage side of a rectifier (7.1 kV) and electrically isolated from the other parts of the furnace. The temperature in the centre of the arc reached about 20,000°C. The arc was surrounded by a sharply decreasing coaxial temperature field, and the temperature at the wall of the electrode was about 600°C. The residence time of the methane in the arc furnace was 5 ms (milliseconds) and a reaction mixture (M1) comprising acetylene, hydrogen, ethylene, soot and unreacted methane was thus produced continuously. At the end of the arc furnace, the reaction mixture (M1) was still at a temperature of about 1,800°C, and then was immediately withdrawn continuously out of the arc furnace and quenched by water with a quench rate of 10⁶ °C/s to about 200°C. The reaction mixture (M1) thus obtained essentially comprised acetylene, hydrogen and ethylene.

In stage (2) of the process, the reaction mixture (M1) thus obtained was then contacted with hydrogen in the presence of the hydrogenation catalyst (C1) prepared in Example 1 (1). The contacting was carried out by continuously introducing the reaction mixture (M1) thus obtained and hydrogen into a reactor (R1) containing the hydrogenation catalyst (C1) at a temperature of 150°C under an absolute pressure of 0.5 MPa, so as acetylene and ethylene were continuously hydrogenated into ethane. Thus, in the reactor (R1), there continuously formed a reaction mixture (M2) essentially comprising ethane and unreacted acetylene, ethylene and hydrogen, and some higher alkanes, in particular C₄ alkanes. The reaction mixture (M2) was then continuously withdrawn out of the reactor (R1) and submitted to a fractionation by change of the physical state of said mixture, in particular by cooling and selective liquefaction, so as to separate and to recycle the unreacted acetylene, ethylene and hydrogen continuously into the reactor (R1), and thus to form a reaction mixture (M2) essentially; comprising ethane and some higher alkanes, in particular C₄ alkanes.

In stage (3) of the process, the reaction mixture (M2) thus obtained was then continuously introduced into a reactor (R2) heated at 150°C, under an absolute pressure of 2 MPa and containing the catalyst (C2) prepared in Example 1 (2), so as ethane was continuously contacted with the catalyst (C2). Thus, by simultaneous ethane self- and cross-metathesis reactions, in the reactor (R2) there continuously formed a reaction mixture (M3) comprising methane and a mixture of the (C₃₊) alkanes, in particular comprising propane, butanes, pentanes and hexanes, and unreacted ethane. The reaction mixture (M3) was withdrawn continuously out of the reactor (R2).

In stage (4) of the process, the reaction mixture (M3) thus obtained was continuously submitted to a fractionation so as to separate and to isolate the mixture of the (C₃₊) alkanes in liquid form. The fractionation was carried out by continuously introducing the reaction mixture (M3) into a fractionation zone (F1), comprising changing the physical state of said reaction mixture, in particular by cooling and selective liquefaction. The mixture of the (C₃₊) alkanes was continuously withdrawn out of the fractionation zone (F1) and recovered in the form of a liquid mixture, essentially comprising propane, butanes, pentanes and hexanes. Simultaneously, in the fractionation zone (F1), the unreacted ethane was continuously separated from the reaction mixture (M3) and recycled into the reactor (R2) of stage (3) wherein the simultaneous ethane self- and cross-metathesis reactions were continued developing towards the production of the mixture of the (C₃₊) alkanes. Furthermore, in the fractionation zone (F1), the unreacted methane also was continuously separated from the reaction mixture (M3) and recycled into the arc furnace of stage (1) for continuing developing the production of acetylene.

### Example 2: conversion of methane into a liquid mixture of the (C₄₊) alkanes.

In a preliminary stage, methane was continuously separated from a natural gas containing methane and 10 % in volume of ethane and C₃ to C₆ alkanes (i.e. a mixture of the (C₂₋₆) alkanes), and having been previously submitted to a purification stage for removing poisons, in particular hydrogen sulfide, carbonyl sulfide, mercaptans, water and carbon dioxide. The preliminary stage comprised continuously fractionating the natural gas so as to separate, to isolate and to recover (a) methane and (b) the mixture of the (C₂₋₆) alkanes. The fractionation of the natural gas was carried out continuously by changing the physical state of the natural gas, in particular by cooling and selective liquefaction.

Stage (1) of the process was performed exactly as in stage (1) of Example 1 (3), apart from using the methane recovered in the preliminary stage.

Stage (2) of the process was performed exactly as in stage (2) of Example 1 (3), apart from using the reaction mixture (M1) obtained in present stage (1).

Stage (3) of the process was performed exactly as in stage (3) of Example 1 (3), apart from continuously introducing into the reactor (R2) the reaction mixture (M2) obtained in present stage (2) and simultaneously the mixture of the (C₂₋₆) alkanes separated from the natural gas in the preliminary stage. Thus, by simultaneous ethane self- and cross-metathesis reactions, in the reactor (R2) there continuously formed a reaction mixture (M3) comprising methane, a mixture of the (C₄₊) alkanes, in particular comprising butanes, pentanes, hexanes and heptanes, and unreacted ethane and propane. The reaction mixture (M3) was withdrawn continuously out of the reactor (R2).

In stage (4) of the process, the reaction mixture (M3) thus obtained was continuously submitted to a fractionation so as to separate and to isolate the mixture of the (C₄₊) alkanes in liquid form. The fractionation was carried out by continuously introducing the reaction mixture (M3) into a fractionation zone (F1), comprising changing the physical state of said reaction mixture, in particular by cooling and selective liquefaction. The mixture of the (C₄₊) alkanes was continuously withdrawn out of the fractionation zone (F1) and recovered in the form of a liquid mixture, essentially comprising butanes, pentanes, hexanes and heptanes. Simultaneously, in the fractionation zone (F1), the unreacted ethane and propane were continuously separated from the reaction mixture (M3) and recycled into the reactor (R2) of stage (3) wherein the simultaneous ethane self- and cross-metathesis reactions were continued developing towards the production of the mixture of the (C₄₊) alkanes. Furthermore, in the fractionation zone (F1), the unreacted methane also was continuously separated from the reaction mixture (M3) and recycled into the arc furnace of stage (1) for continuing developing the production of acetylene.

### Example 3: conversion of natural gas into a liquid mixture of the (C₅₊) alkanes.

Stage (1) of the process was performed exactly as in stage (1) of Example 1 (3), apart from using instead of methane, a natural gas containing methane and 10 % in volume of ethane and C₃ to C₆ alkanes (i.e. a mixture of the (C₂₋₆) alkanes), and having been previously submitted to a purification stage for removing poisons, in particular hydrogen sulfide, carbonyl sulfide, mercaptans, water and carbon dioxide. A reaction mixture (M1) comprising acetylene, hydrogen, ethylene, soot and unreacted methane and (C₂₋₆) alkanes was thus continuously obtained and submitted to a fractionation by change of the physical state of said reaction mixture, in particular by cooling and selective liquefaction, so as to separate and to recycle the unreacted methane continuously into the arc furnace, and thus to form a reaction mixture (M1) substantially free from methane and essentially comprising acetylene, ethylene, hydrogen and the unreacted (C₂₋₆) alkanes.

Stage (2) of the process was performed exactly as in stage (2) of Example 1 (3), apart from using the reaction mixture (M1) obtained in present stage (1).

Stage (3) of the process was performed exactly as in stage (3) of Example 1 (3), apart from using the reaction mixture (M2) obtained in present stage (2). Thus, in the reactor (R2) there continuously formed a reaction mixture (M3) comprising methane, a mixture of the (C₅₊) alkanes, in particular comprising pentanes, hexanes, heptanes and octanes, and unreacted ethane, propane and butanes. The reaction mixture (M3) was withdrawn continuously out of the reactor (R2).

In stage (4) of the process, the reaction mixture (M3) thus obtained was continuously submitted to a fractionation so as to separate and to isolate the mixture of the (C₅₊) alkanes in liquid form. The fractionation was carried out by continuously introducing the reaction mixture (M3) into a fractionation zone (F1), comprising changing the physical state of said reaction mixture, in particular by cooling and selective liquefaction. The mixture of the (C₅₊) alkanes was continuously withdrawn out of the fractionation zone (F1) and recovered in the form of a liquid mixture, essentially comprising pentanes, hexanes, heptanes and octanes. Simultaneously, in the fractionation zone (F1), the unreacted ethane, propane and butanes were continuously separated from the reaction mixture (M3) and recycled into the reactor (R2) of stage (3) wherein the simultaneous ethane self- and cross-metathesis reactions were continued developing towards the production of the mixture of the (C₅₊) alkanes. Furthermore, in the fractionation zone (F1), the unreacted methane also was continuously separated from the reaction mixture (M3) and recycled into the arc furnace of stage (1) for continuing developing the production of acetylene.

## Claims

1. Process for converting methane into a liquid mixture of (C₃₊) alkanes having 3 carbon atoms and more, preferably (C₄₊) alkanes having 4 carbon atoms and more, especially (C₅₊) alkanes having 5 carbon atoms and more, **characterised in that** it comprises:
- a stage (1) comprising converting methane into acetylene, carried out by submitting methane to a thermal cracking reaction, so as to form a reaction mixture (M1) comprising acetylene and hydrogen, and optionally by fractionating said reaction mixture (M1) so as to separate the acetylene,
- a stage (2) comprising converting acetylene into ethane, carried out by contacting the acetylene or the reaction mixture (M1) previously obtained with hydrogen in the presence of a hydrogenation catalyst (C1), so as to form a reaction mixture (M2) comprising ethane, and optionally by fractionating said reaction mixture (M2) so as to separate the ethane,
- a stage (3) comprising simultaneous ethane self- and cross-metathesis reactions, carried out by contacting the ethane or the reaction mixture (M2) previously obtained with a metal catalyst (C2) capable of producing, in contact with alkane, reactions involving the splitting and recombining of carbon-carbon and/or carbon-hydrogen and/or carbon-metal bonds, so as to form a reaction mixture (M3) comprising methane and a mixture of the (C₃₊), preferably the (C₄₊), especially the (C₅₊) alkanes, and
- a stage (4) comprising fractionating the reaction mixture (M3) so as to separate and to isolate said mixture of the (C₃₊), preferably the (C₄₊), especially the (C₅₊) alkanes, in liquid form.

2. Process according to claim 1, **characterised in that** in stage (1), methane is used either alone or in the form of a mixture of methane, preferably in a major molar proportion, with one or more other alkane(s) preferably selected from the other alkanes present in natural gas, particularly the (C₂₊) alkanes having 2 carbon atoms and more, especially in the form of natural gas.

3. Process according to claim 2, **characterised in that** prior to stage (1), it comprises a purification stage comprising removing from the methane or from the natural gas, one or more poisons capable of deactivating the hydrogenation catalyst (C1) and/or the metal catalyst (C2), and/or one or more impurities, particularly nitrogen.

4. Process according to claim 2 or 3, **characterised in that** prior to stage (1), it comprises a preliminary stage comprising fractionating the components of the natural gas so as to separate and to isolate (a) the methane from (b) the rest of the natural gas essentially comprising a mixture of the (C₂₊) alkanes having 2 carbon atoms and more.

5. Process according to any one of claims 1 to 4, **characterised in that** in stage (1), the thermal cracking reaction is performed by submitting methane to a temperature higher than 730°C, preferably higher than 1,000°C, particularly higher than 1,200°C.

6. Process according to any one of claims 1 to 5, **characterised in that** in stage (1), the thermal cracking reaction is performed with a reaction time selected from 0.1 to 50 ms, preferably from 0.1 to 30 ms, particularly from 0.1 to 10 ms.

7. Process according to any one of claims 1 to 6, **characterised in that** in stage (1), the thermal cracking reaction is stopped by quenching the reaction mixture (M1) obtained to a temperature selected from 50 to 300°C; preferably from 80 to 250°C, in particular with a quench rate selected from 10⁴ to 10⁸, preferably from 10⁵ to 10⁷ °C/s.

8. Process according to any one of claims 1 to 7, **characterised in that** in stage (1), the thermal cracking reaction is performed by a high-temperature process selected from partial combustion processes, electrothermic processes and high-temperature pyrolysis processes.

9. Process according to claim 8, **characterised in that** the partial combustion profess comprises (i) preheating methane and oxygen preferably separately, in particular at a temperature selected from 500 to 700°C, (ii) passing the methane and the oxygen thus preheated through a mixing zone and a burner into a cracking reaction zone wherein they are partially ignited, preferably at a temperature above 1,000°C, in particular above 1,200°C, particularly with a reaction time selected from 0.1 to 50 ms, so as to form the reaction mixture (M1), and (iii) quenching said reaction mixture (M1) when leaving the cracking reaction zone, preferably by water or a hydrocarbon liquid or an oil, particularly to a temperature selected from 50 to 300°C, especially with a quench rate selected from 10⁴ to 10⁸ °C/s.

10. Process according to claim 8, **characterised in that** the electrothermic process comprises (i) injecting methane into an arc furnace, preferably under an absolute pressure chosen from 0.05 to 0.15 MPa, (ii) maintaining the methane in the arc furnace during a reaction time selected from 0.1 to 50 ms, so as to form the reaction mixture (M1), and (iii) quenching said reaction mixture (M1) when leaving the arc furnace, preferably with the help of water or a hydrocarbon liquid or an oil, preferably to a temperature selected from 50 to 300°C, particularly with a quench rate selected from 10⁴ to 10⁸ °C/s.

11. Process according to claim 8, **characterised in that** the high-temperature pyrolysis process comprises (i) cracking methane with a reaction time preferably selected from 0.1 to 50 ms, in a refractory oven previously heated by combustion gases to a temperature preferably selected from 1,000 to 3,000°C, so as to form the reaction mixture (M1), and (ii) quenching said reaction mixture (M1) outside said refractory oven to a temperature preferably selected from 50 to 300°C, in particular with a quench rate selected from 10⁴ to 10⁸ °C/s.

12. Process according to any one of claims 1 to 11, **characterised in that** the reaction mixture (M1) comprising acetylene, hydrogen, and optionally ethylene and unreacted methane or unreacted natural gas, preferably obtained in stage (1) by a thermal cracking reaction carried out by an electrothermic process, is directly used in stage (2) for converting acetylene and optionally ethylene into ethane.

13. Process according to any one of claims 1 to 11, **characterised in that** the reaction mixture (M1) comprising acetylene, hydrogen and optionally ethylene and unreacted methane or unreacted natural gas, is submitted, prior to stage (2), to a fractionation comprising separating and preferably isolating the acetylene optionally in mixture with the ethylene and/or the unreacted methane or the unreacted natural gas, and optionally with the hydrogen, particularly in molar proportions with regard to acetylene identical to or lower than those in said reaction mixture (M1).

14. Process according to any one of claims 1 to 11 and 13, **characterised in that** the reaction mixture (M1) comprising acetylene, hydrogen and optionally ethylene and unreacted methane or unreacted natural gas, is submitted, prior to stage (2), to a fractionation comprising separating from said reaction mixture (M1) at least a portion of the unreacted methane or the unreacted natural gas, preferably recycled to the thermal cracking reaction in stage (1).

15. Process according to any one of claims 1 to 11, 13 and 14, **characterised in that** the reaction mixture (M1) comprising oxygen-containing products selected from carbon monoxide, carbon dioxide and unreacted oxygen, particularly obtained by a thermal cracking reaction carried out by a partial combustion process, is submitted, prior to stage (2), to a fractionation comprising separating and eliminating said oxygen-containing products from said reaction mixture (M1).

16. Process according to any one of claims 1 to 15, **characterised in that** in stage (2), the acetylene is used either alone or in the form of a mixture of acetylene, preferably in a major molar proportion, with ethylene and/or one or more alkanes, particularly with the ethylene and/or the unreacted methane or the unreacted natural gas present in the reaction mixture (M1), especially in molar proportions with regard to acetylene identical to or lower than those in said reaction mixture (M1).

17. Process according to any one of claims 1 to 16, **characterised in that** in stage (2), the acetylene is used either alone or in the form of a mixture of acetylene, preferably in a major molar proportion, with ethylene and/or one or more alkanes, particularly the ethylene and/or the unreacted methane or the unreacted natural gas present in the reaction mixture, especially in molar proportions with regard to acetylene identical to or lower than those in said reaction mixture (M1).

18. Process according to any one of claims 1 to 17, **characterised in that** in stage (2), the hydrogen is used either alone or in the form of a mixture of hydrogen with acetylene, in particular with the reaction mixture (M1).

19. Process according to any one of claims 1 to 18, **characterised in that** in stage (2), the hydrogenation catalyst (C1) is selected from supported metal catalysts and supported metal sulfide catalysts, preferably from supported transition metal catalysts and supported transition metal sulfide catalysts.

20. Process according to claim 19, **characterised in that** the supported transition metal catalyst comprises one, two or more transition metals, preferably selected from chromium, molybdenum, tungsten, rhenium, iron, ruthenium, osmium, cobalt, rhodium, iridium, nickel, palladium, platinum, silver, gold, copper and zinc, particularly from chromium, rhenium, iron, ruthenium, cobalt, rhodium, iridium, nickel, palladium, platinum, silver, gold and copper, especially from rhenium, rhodium, iridium, nickel, palladium and platinum.

21. Process according to claim 19, **characterised in that** the supported transition metal sulfide comprises one, two or more transition metal sulfides selected from the sulfides of chromium, molybdenum, tungsten, rhenium, iron, ruthenium, osmium, cobalt, rhodium, iridium, nickel, palladium, platinum, copper and zinc, particularly from the sulfides of chromium, iron, rhenium, ruthenium, cobalt, rhodium, iridium, nickel, palladium, platinum and copper.

22. Process according to any one of claims 1 to 21, **characterised in that** the hydrogenation catalyst (C1) comprises a support selected from metal oxides, refractory oxides, metals, carbon and molecular sieves, preferably from silica, alumina, silica-alumina, aluminium silicate, carbon, zeolites, clays, titanium oxide, cerium oxide, magnesium oxide, niobium oxide, tantalum oxide and zirconium oxide, more particularly from silica, alumina, silica-alumina and carbon.

23. Process according to any one of claims 1 to 22, **characterised in that** in stage (2), the contacting is performed at a temperature selected from 0 to 400°C, preferably from 20 to 300°C.

24. Process according to any one of claims 1 to 23, **characterised in that** in stage (2), the contacting is performed under an absolute total pressure selected from 0.1 to 10 MPa, preferably from 0.1 to 1.0 MPa.

25. Process according to any one of claims 1 to 24, **characterised in that** in stage (2), the contacting is performed in the presence of one or more inert agents, in particular liquid or gaseous, preferably selected from inert gases, particularly from nitrogen, helium and argon, and from alkanes, particularly from methane, ethane and alkanes of the natural gas, especially from the unreacted methane or the unreacted natural gas present in the reaction mixture (M1).

26. Process according to any one of claims 1 to 25, **characterised in that** in stage (2), the contacting is performed in a reaction zone selected from tubular or multi-tubular reactors, slurry reactors, fluidised bed reactors, mechanically agitated bed reactors, fluidised and mechanically agitated bed reactors, fixed bed reactors, moving bed reactors and circulating bed reactors.

27. Process according to any one of claims 1 to 26, **characterised in that** the reaction mixture (M2) comprising ethane and optionally unreacted acetylene, ethylene and hydrogen, and optionally one or more other alkanes, preferably selected from methane and natural gas, is used directly in stage (3).

28. Process according to any one of claims 1 to 26, **characterised in that** the reaction mixture (M2) comprising ethane and optionally unreacted acetylene, ethylene and hydrogen, and optionally one or more other alkanes preferably selected from methane and natural gas, is submitted, prior to stage (3), to a fractionation comprising separating and preferably isolating the ethane or a mixture of the ethane with said one or more other alkanes preferably selected from methane and natural gas, and optionally with the hydrogen.

29. Process according to any one of claims 1 to 26, and 28, **characterised in that** the reaction mixture (M2) comprising ethane and optionally unreacted acetylene, ethylene and hydrogen, and optionally one or more other alkanes, is submitted, prior to stage (3), to a fractionation comprising separating from said reaction mixture (M2) the unreacted acetylene, ethylene and optionally hydrogen, preferably recycled in stage (2).

30. Process according to any one of claims 1 to 29, **characterised in that** in stage (3), the ethane is used either alone or in the form of a mixture of ethane, preferably in a major molar proportion, with one or more other higher alkanes preferably selected from the other higher alkanes of the natural gas, particularly from the mixture of the (C₂₊) alkanes having 2 carbon atoms and more of the natural gas, especially in molar proportions with regards to ethane similar or identical to those existing in the natural gas.

31. Process according to any one of claims 1 to 29, **characterised in that** in stage (3), the ethane or the reaction mixture (M2) obtained in stage (2) is used in mixture or in combination with a mixture of the (C₂₊) alkanes having 2 carbon atoms and more, obtained by fractionation of natural gas in a preliminary stage from which methane is derived and used in stage (1).

32. Process according to claim 31, **characterised in that** in stage (3), the ethane or the reaction mixture (M2) obtained in stage (2) and the mixture of the (C₂₊) alkanes are contacted either separately or simultaneously, preferably in the form of a pre-mixture, with the metal catalyst (C2).

33. Process according to any one of claims 1 to 32, **characterised in that** the metal catalyst (C2) is selected from alkane metathesis catalysts, preferably from supported metal clusters and from metal hydrides, organometallic compounds and organometallic hydrides, in particular supported on, and especially grafted onto a solid support.

34. Process according to claim 33, **characterised in that** the supported metal cluster comprises one or more metals of Groups 4, 5 and/or 6 of the Periodic Table of the Elements, preferably tantalum.

35. Process according to claim 33 or 35, **characterised in that** the supported metal cluster comprises a solid support and a metal cluster, preferably bonding between the metals of the metal cluster, and bonding between the metal cluster and the solid support.

36. Process according to claim 35, **characterised in that** the metal atom of the metal cluster is bonded to at least one other metal of the metal cluster, preferably to six or fewer metal atoms of the metal cluster.

37. Process according to any one of claims 33 to 36, **characterised in that** the supported metal cluster comprises a solid support chosen from a metal oxide, a refractory oxide, a molecular sieve, a zeolite and a metal phosphate.

38. Process according to claim 33, **characterised in that** the catalyst chosen from metal hydrides, organometallic compounds and organometallic hydrides, comprises at least one metal chosen from lanthanides, actinides and metals of Groups 2 to 12, preferably transition metals of Groups 3 to 12 of the Periodic Table of the Elements.

39. Process according to claim 38, **characterised in that** the catalyst comprises at least one metal chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium, preferably from yttrium, titanium, zirconium, hafnium, vanadium, niobium, tantalum, chromium, molybdenum, tungsten, rhenium, ruthenium and platinum, and more particularly from niobium, tantalum, molybdenum, tungsten and rhenium.

40. Process according to any one of claims 33, 38 and 39, **characterised in that** the catalyst is supported on, or grafted onto a solid support chosen from metal or refractory oxides, sulfides, carbides, nitrides and salts, and from carbon, mesoporous materials; organic/inorganic hybrid materials, metals and molecular sieves.

41. Process according to any one of claims 1 to 40, **characterised in that** stage (3) is performed at a temperature chosen from 20 to 400°C, preferably from 50 to 350°C, particularly from 70 to 300°C.

42. Process according to any one of claims 1 to 41, **characterised in that** stage (3) is performed under an absolute total pressure chosen from 0.1 to 50 MPa, preferably from 0.1 to 30 MPa, particularly from 0.1 to 20 MPa.

43. Process according to any one of claims 1 to 41, **characterised in that** stage (3) is performed in the presence of at least one inert agent, preferably an inert gas, in particular selected from nitrogen, helium and argon, and/or in the presence of hydrogen, preferably the hydrogen of the reaction mixture (M1), particularly under a partial pressure of hydrogen chosen from 0.1 kPa to 10 MPa, preferably from 1 kPa to 1 MPa.

44. Process according to any one of claims 1 to 43, **characterised in that** stage (3) is performed in a reaction zone (Z 1) which is either distinct and separate from a fractionation zone (F1) wherein stage (4) is performed, or arranged in a part of said fractionation zone (F1), preferably in at least one distillation column reactor.

45. Process according to any one of claims 1 to 44, **characterised in that** stage (3) is performed in a reaction zone comprising at least one reactor chosen from static reactors, recycling reactors and dynamic reactors.

46. Process according to any one of claims 1 to 45, **characterised in that** stage (3) is performed in a reaction zone comprising at least one reactor chosen from tubular or multi-tubular reactors, distillation column reactors, slurry reactors, fluidised bed reactors, mechanically agitated bed reactors, fluidised and mechanically agitated bed reactors, fixed bed reactors, moving bed reactors and circulating bed reactors.

47. Process according to any one of claims 1 to 46, **characterised in that** stage (3) is performed (i) by continuously introducing the ethane and optionally a mixture of the (C2+) alkanes pre-mixed or separated, into a reaction zone (Z1) containing the catalyst (C2), so as to form the reaction mixture (M3), and (ii) by continuously withdrawing at least a part of the reaction mixture (M3) out of the reaction zone (Z1), so as to subject said part of the reaction mixture (M3) to stage (4) for separating the mixture of the (C₃₊), preferably the (C₄₊), especially the (C₅₊) alkanes and isolating said mixture from the rest of the reaction mixture (M3) which is preferably returned into the reaction zone (Z1).

48. Process according to any one of claims 1 to 47, **characterised in that** stage (4) comprises one or more methods of fractionation of the reaction mixture (M3) chosen from
(i) fractionation by change of physical state, preferably of gaseous/liquid phase of the mixture, particularly by distillation or by condensation, especially by means of distillation/condensation column or tower, (ii) fractionation by molecular filtration, preferably by means of semi-permeable and selective membrane, (iii) fractionation by adsorption, preferably by means of molecular sieve or any other adsorbent, (iv) fractionation by absorption, preferably by means of absorbing oil, (v) fractionation by cryogenic expansion, preferably by means of expansion turbine, and (vi) fractionation by compression, preferably by means of gas compressor.

49. Process according to any one of claims 1 to 48, **characterised in that** stage (4) is performed by a double or multiple fractionation of the reaction mixture (M3) of an identical or different type, comprising separating and isolating (a) the mixture of the (C₃₊), preferably the (C₄₊), especially the (C₅₊) alkanes, in liquid form, (b) the methane preferably recycled into stage (1) for the methane conversion into acetylene, and optionally unreacted ethane and where applicable unreacted propane and/or butanes, preferably recycled into stage (3) for the simultaneous ethane self- and cross-metathesis reactions.
